# EUROPEAN PATENT SPECIFICATION

(11) **EP 3 728 313 B1**
(45) Date of publication and mention of the grant of the patent: **02.08.2023**
(21) Application number: 18829861.6
(22) Date of filing: 21.12.2018
(51) Int. Cl.: C07K 16/28, A61K 45/06, A61P 35/00, A61K 39/395

(54) **COMBINATION ANTI CANCER THERAPY WITH AN IAP ANTAGONIST AND AN ANTI PD-1 MOLECULE**
KOMBINATIONSTHERAPIE GEGEN KREBS MIT EINEM IAP-ANTAGONISTEN UND EINEM ANTI-PD-1-MOLEKÜL
POLYTHÉRAPIE CONTRE LE CANCER FAISANT INTERVENIR UN ANTAGONISTE D'IAP ET UNE MOLÉCULE ANTI PD-1

(30) Priority: 21.12.2017 WO PCT/IB2017/001595
(43) Date of publication of application: 28.10.2020
(73) Proprietor: Debiopharm International S.A., 1006 Lausanne (CH)
(72) Inventor: VUAGNIAUX, Grégoire, 1005 Lausanne (CH); WIEDEMANN, Norbert, 1006 Lausanne (CH); GAVILLET, Bruno, 01220 Grilly (FR); SZYLDERGEMAJN ALTMAN, Sergio Adrian, 1162 Saint Prex (CH)
(74) Representative: Hoffmann Eitle
(86) International application number: PCT/EP2018/086606
(87) International publication number: WO 2019/122337

(56) References cited:
- Anonymous: "A Dose-Finding Study of the Second Mitochondrial Activator of Caspases (SMAC) Mimetic Debio 1143 When Given in Combination With Avelumab to Participants With Advanced Solid Malignancies and to Participants With Advanced or Metastatic Non-Small Cell Lung Cancer (NSCLC) After Platinum-Based Therapy", clinical trials.gov, 18 December 2017 (2017-12-18), pages 1-8, XP055502607, Retrieved from the Internet: URL:https://clinicaltrials.gov/ct2/history /NCT03270176?V_7=View#StudyPageTop [retrieved on 2018-08-28]
- Anonymous: "NCT02587962:Dose-escalation Study of Birinapant and Pembrolizumab in Solid Tumors", , 8 November 2017 (2017-11-08), pages 1-8, XP055503207, Retrieved from the Internet: URL:https://clinicaltrials.gov/ct2/history /NCT02587962?V_4=View#StudyPageTop [retrieved on 2018-08-29]
- Anonymous: "NCT02890069: Phase Ib, Open-label, Multi-center Study to Characterize the Safety, Tolerability and Pharmacodynamics (PD) of PDR001 in Combination With LCL161, Everolimus (RAD001) or Panobinostat (LBH589)", clinical trials.gov, 1 December 2017 (2017-12-01), pages 1-9, XP055502777, Retrieved from the Internet: URL:https://clinicaltrials.gov/ct2/history /NCT02890069?V_8=View#StudyPageTop [retrieved on 2018-08-28]
- SHAWN T. BEUG ET AL: "Smac mimetics synergize with immune checkpoint inhibitors to promote tumour immunity against glioblastoma", NATURE COMMUNICATIONS, vol. 8, 15 February 2017 (2017-02-15), XP055482024, DOI: 10.1038/ncomms14278
- Yvonne Bo: "Role of Smac in Lung Carcinogenesis and Therapy", , 1 July 2017 (2017-07-01), page e107165, XP055502783, United States DOI: 10.1371/journal.pone.0107165 Retrieved from the Internet: URL:http://www.dtic.mil/dtic/tr/fulltext/u 2/1050348.pdf [retrieved on 2018-08-28]
- CONOR J KEARNEY ET AL: "PD-L1 and IAPs co-operate to protect tumors from cytotoxic lymphocyte-derived TNF", CELL DEATH AND DIFFERENTIATION., vol. 24, no. 10, 30 June 2017 (2017-06-30) , pages 1705-1716, XP055502801, GB ISSN: 1350-9047, DOI: 10.1038/cdd.2017.94

## Description

### FIELD OF THE INVENTION

The present invention relates to the use of an IAP antagonist for enhancing the immunogenicity of the microenvironment of a subject's cancer prior to a treatment of the subject with an anti-PD-1 molecule.

### BACKGROUND OF THE INVENTION

A number of cancer types comprise cases in which components of the extrinsic or intrinsic cell death pathways are genetically altered. This can involve overexpression of FAS-associated via death domain (FADD) or inhibitor of apoptosis proteins (IAP), or a lack of expression of functional caspases. The result can be resistance to cell death, a hallmark of cancer. Hoadley et al. (2014) Cell 158: 929-44; The Cancer Genome Atlas Network (2015) Nat 517: 576-82; Eytan et al. (2016) Cancer Res 76: 5442-54; Hanahan and Weinberg (2011) Cell 144: 646-74.

A succinct description of the extrinsic and intrinsic death pathways is found in Derakhshan et al. (2017) Clin Cancer Res 23: 1379-87. Briefly, the extrinsic pathway begins at a cell surface receptor. It is triggered by the binding of death ligands such as Fas ligand (FasL), TNFα or TRAIL to their respective receptors (i.e., Fas, TNFR1, TRAILR1/DR4, TRAIL2/DR5) on the extracellular side. As a consequence, FADD binds to the receptors on the intracellular side, and procaspase 8 binds to the receptor-bound FADD to constitute the death-inducing signaling complex (DISC). This is followed by activation of caspase 8 and then caspase 3, leading to apoptosis. The extrinsic pathway may also cause necroptotic death, involving FADD, RIP kinases and mixed lineage kinase domainlike protein (MLKL).

The intrinsic pathway begins with an insult to mitochondria which results in a release into the cytoplasm of proapoptotic proteins such as cytochrome c and second mitochondriaderived activator of caspases (SMAC). Cytochrome c binds apoptotic protease activating factor (APAF1), forming the apoptosome complex. The complex binds to procaspase 9, which is activated and in turn activates procaspase 3. SMAC binds to and causes the degradation or inhibition of IAP family proteins, including cellular IAP1 (clAP1), cellular IAP2 (clAP2) and X-linked IAP (XIAP).

IAP are proteins that are defined by the presence of one to three baculoviral IAP repeat (BIR) domains. Human cells express 8 different IAP, of which XIAP, clAP1 and clAP2 were shown to inhibit caspase-induced apoptosis and RIP kinase-mediated necroptosis. Salvesen and Duckett (2002) Nat Rev Mol Cell Biol 3: 401-10. Of the latter IAP, only XIAP is capable of directly binding to caspases and inhibiting their function. Derakhshan et al. (Clin Cancer Res. 2017 Mar 15;23(6):1379-1387. doi: 10.1158/1078-0432.CCR-16-2172. Epub 2016 Dec 30). cIAP1, cIAP2 and XIAP contain a so-called RING domain that has E3 ubiquitin ligase activity. The anti-apoptotic effect of clAP1 and clAP2 is mediated by their ubiquitin ligase activity.

SMAC is a dimeric protein that contains at its amino terminus the peptide sequence Ala-Val-Pro-Ile (AVPI) which sequence is mediating the binding of the protein to BIR domains of IAP. Peptidomimetics were developed that mimic the latter peptide sequence thereby duplicating SMAC's ability to bind XIAP, clAP1 and clAP2 (referred to herein as "SMAC mimetics"). The SMAC mimetics prevent XIAP from interacting with caspases. Regarding clAP1 and clAP2, the SMAC mimetics activate the E3 ubiquitin ligase activity of the lAPs, causing their auto-ubiquitylation and elimination by proteasomal degradation.

In addition to their inhibitory effects on apoptosis, lAPs also influence a multitude of other cellular processes, such as ubiquitin-dependent signaling events that regulate activation of NF-κB transcription factor, which drives the expression of genes important for inflammation, immunity, cell migration, and cell survival. Gyrd-Hansen and Meier (2010) Nat Rev Cancer 10: 561-74. Cellular lAPs are critical in the canonical pathway of NF-κB activation. Derakhshan et al. (2017). Binding of TNFα to TNFR1 results in recruitment of TNF receptor 1-associated via death domain (TRADD) and TNF receptor-associated factor 2 (TRAF2) to TNFR1. RIP1 and cIAP1/2 are then recruited to the active complex. Cellular lAP-mediated ubiquitination of RIP1 eventually results in the phosphorylation of the inhibitor of NF-κB kinase IKKβ which phosphorylates the inhibitory NF-kB subunit Ikβ. Ikβ is then degraded, liberating NF-kB subunits p50 and RELA which combine to form active transcription factor NF-κB. This engagement of TNFR1 prevents its apoptotic or necroptotic signaling. IAP-dependent regulation of NF-κB signaling pathways has a major impact on the function of the immune system, affecting both innate and adaptive immunity. Beug et al. (2012) Trends Immunol 33: 535-45. Thus, lAPs have been demonstrated to regulate the function of several immune cell types relevant for anti-tumor immune responses including antigen-presenting cells, lymphocytes, and natural killer cells.

Cellular lAPs are also responsible for the ubiquitination of NF-κB-inducing kinase NIK, resulting in its proteasomal degradation. Derakhshan et al. (2017). In the absence of lAPs, i.e., in the presence of an IAP antagonist such as a SMAC mimetic, NIK accumulates and phosphorylates IKKα which phosphorylates inactive NF-κB subunit p100. The subunit is cleaved to active subunit p52, which combines with RELB to form an active NF-kB transcription factor. This noncanonical activation of NF-kB is crucial for the modulation of innate and adaptive immunity by cytokine production. Chesi et al. (2016) Nat Med, 22:1411-20, and references cited therein. IAP inhibitor LBW242 was shown to increase anti-tumor immune responses by inducing T-cell proliferation and co-stimulation in the context of a primary T-cell receptor stimulus, leading to increased T-cell activation, and enhanced efficacy in a prophylactic cancer vaccine model. Dougan et al. (2010) J Exp Med 207: 2195-206. IAP inhibitors BV6 and birinapant were shown to modulate the function of antigen-presenting cells, e.g. by inducing dendritic cell maturation, or by converting pro-tumoral type-II macrophages into pro-inflammatory type-I macrophages. Muller-Sienerth et al. (2011) PLoS One 6: e21556; Knights et al. (2013) Cancer Immunol Immunother 62: 321-35; Lecis et al. (2013) Cell Death Dis 4: e920. Moreover, IAP inhibition increases the susceptibility of tumor cells towards natural killer cell- or T cellmediated effector mechanisms granzyme B and perforin. Brinkmann et al. (2014) Leuk Lymphoma 55: 645-51; Nachmias et al. (2007) Eur J Immunol 37: 3467-76. In addition, IAP inhibitors might also contribute to immune system regulation by modulating the expression of immune checkpoint molecules on immune cells. Knights et al. (2013); Pinzon-Ortiz et al. (2016) Cancer Res 76 (14 Suppl): abstract 2343. It is noted that in the absence of lAPs, i.e., in the presence of an IAP antagonist such as a SMAC mimetic, TNFR1 is no longer engaged in canonical NF-kB activation, rendering cells sensitive to TNFα-mediated apoptosis.

Typically, immune destruction of tumor cells is inefficient. It now appears that this is because cancer patients do not have a significant reservoir of T cells capable of destroying the tumor and/or because cells of the adaptive and innate immune systems are held in check or are neutralized by pathways that inhibit their activation or their effector functions. Instrumental in this suppression are so-called immune checkpoint molecules. Several such checkpoint molecules have been identified over the last twenty years. The prototypical molecule of this type is the cytotoxic T lymphocyte antigen 4 (CTLA-4). Blocking this molecule was found to result in tumor regression in murine models. Leach et al. (1996) Science 271: 1734-36. CTLA-4 is expressed on activated T cells, predominantly on CD4 cells, and limits T cell responses by interfering with the activity of master T cell costimulator CD28. CTLA-4 and CD28 share ligands CD80 and CD86, whereby CTLA-4 outcompetes CD28 due to its higher affinity for the latter ligands. Linsley et al. (1994) Immunity 1: 793-801.

Like CTLA-4, immune checkpoint molecule PD-1 is expressed on activated T cells. Parry et al. (2005) Mol Cell Biol 25: 9543-53. It also activates phosphatases SHP2 and PP2A. Engagement of PD-1 is thought to directly interfere with TCR-mediated effector functions and increase T cell migration. The checkpoint molecule is believed to exert its function primarily in the tumor microenvironment, whereas CTLA-4 acts primarily in secondary lymphoid tissues. Wing et al. (2008) Science 322: 271-5; Peggs et al. (2009) J Exp Med 206: 1717-1725. The two known ligands of PD-1 are PD-L1 and PD-L2. Dong et al. (1999) Nat Med 5: 1365-9; Latchman et al. (2001) Nat Immunol 261-8; Tseng et al. (2001) J Exp Med 193: 839-46. The ligand molecules share homology but are divergently regulated. PD-L1 is induced in activated hematopoietic and epithelial cells by IFNγ (produced by activated T cells and natural killer cells). PD-L2 is found induced in activated dendritic cells and some macrophages. Induction may be predominantly by IL-4. PD-1 knockout mice exhibit late-onset organ-specific inflammation. Nishimura et al. (1999) Immunity 11: 141-51; Science 291: 319-22 (2001). This phenotype is much less severe than that observed in CTLA-4 knockout mice. Correspondingly, clinical immune-related effects of anti-PD-1 therapy tend to be milder than those associated with anti-CTLA-4 therapy. PD-L1 is expressed in many solid tumors, and PD-L2 in certain subsets of B cell lymphomas. PD-1 is highly expressed in tumor-infiltrating lymphocytes. Dong et al. (2002) Nat Med 8: 793-800; Ansell et al. (2015) N Engl J Med 372: 311-9; Amadzadeh et al. (2009) Blood 114: 1537-44; Sfanos et al. (2009) Prostate 69: 1694-1703.

The first human trials of anti-PD-1 therapy employed monoclonal antibody Nivolumab, a fully human IgG4 antibody from Bristol-Myers Squibb/Ono Pharmaceuticals. Objective response rates of 17% for advanced treatment-refractory NSCLC, 20% for RCC and 31% for melanoma were documented. Many of these responses were long-lasting. Overall survival was 9.9, 22.4 and 16.8 months, respectively. Topalian et al. (2012) N Engl J Med 366: 2443-54; J Clin Oncol 32: 1020-30 (2014). To date, Nivolumab has been approved in the U.S., Japan and Europe for the treatment of unresectable or metastatic melanoma, for renal carcinoma (RCC), metastatic or recurrent squamous cell carcinoma of head and neck (SCCHN), metastatic non-small cell lung carcinoma (NSCLC) and Hodgkin lymphoma. Iwai et al. (2017) J Biomed Science 24: 36; Balar and Weber (2017) Cancer Immunol Immunother 66: 551-64. FDA approval for urothelial cancer has also been obtained. Monoclonal anti-PD-1 antibody Pembrolizumab, a humanized IgG4 antibody from Merck has also been approved for metastatic melanoma, metastatic NSCLC (U.S., Japan and Europe) as well as for head & neck cancer and microsatellite instability (MSI) high tumors from agnostic primary site (U.S.). Atezolizumab, another antibody of the IgG1 type from Roche/Genentech, inhibits the ligand PD-L1. It obtained FDA approval for urothelial cancer (bladder cancer) and metastatic NSCLC. Two additional PD-L1 antibodies recently appeared in the market. Durvalumab is a human IgG1k antibody from Medimmune/AstraZeneca that is FDA-approved for locally advanced or metastatic urothelial cancer. Avelumab is a human IgG1 antibody from Merck Serono/Pfizer that has been approved by the FDA for the treatment of metastatic Merkel cell carcinoma and urothelial/bladder cancer. Additional molecules directed to PD-1 are moving through clinical trials. These include humanized IgG4 antibody PDR001 from Novartis, monoclonal antibody IBI-308 from Innovent Biologics, fully-humanized monoclonal antibody cemiplimab (REGN-2810) from Regeneron, humanized IgG4 monoclonal antibody camrelizumab (SHR-1210) from Jiangsu Hengrui Medicine, BGB-A317 monoclonal humanized antibody from BeiGene, monoclonal antibody BCD-100 from Biocad, humanized IgG4K recombinant antibody JS-001 from Shanghai Junshi Biosciences, JNJ-3283 (JNJ-63723283) monoclonal antibody from Johnson & Johnson, monoclonal antibody AMP-514 (now called "MEDI0680") from Amplimmune (now Medimmune [AstraZeneca]), AGEN-2034 by Agenus, humanized monoclonal antibody TSR-042 from AnaptysBio and Tesaro, humanized monoclonal antibody Sym-021 from Symphogen, PF-06801591 antibody from Pfizer, bi-specific tetravalent humanized DART (dual-affinity retargeting) molecule MGD-013 from Macrogenics, MGA-012 humanized monoclonal antibody from Macrogenics, recombinant humanized antibody LZM-009 from Livzon Pharmaceutical, human recombinant monoclonal antibody GLS-010 (AB-122) from Gloria Pharmaceuticals, IgG4 humanized monoclonal antibody genolimzumab (CBT-501) from Walvax Biotechnology, monoclonal antibody B! 754091 from Boehringer Ingelheim and bispecific monoclonal antibody AK-104 from Akeso Biopharma. Additional molecules directed to PD-L1 are also moving through clinical trials. These include monoclonal antibody CX-072 from CytomX Therapeutics, fully humanized recombinant IgG monoclonal antibody WBP3155 (CS-1001) from CStone Pharmaceuticals, humanized IgG4 monoclonal antibody SHR-1316 from Atridia, PD-L1 Inhibitor millamolecule from Bristol-Myers Squibb, human IgG4 antibody BMS-936559 (MDX1105) from Bristol-Myers Squibb, bi-functional fusion protein targeting PD-L1 monoclonal antibody and TGFβ M-7824 (MSB0011359C) from Merck KGaA, monoclonal antibody LY-3300054 from Eli Lilly, nanobody KN-035 from Alphamab, monoclonal antibody FAZ-053 from Novartis, IgG1 antibody CK-301 from TG Therapeutics, oral small molecule CA-170 targeting PD-L1 and V-domain Ig suppressor of T cell activation (VISTA) from Aurigene Discovery. As of 2015, objective response rates for anti-PD-1/PD-L1 therapies had been reported to be 17-40% for melanoma, 10-30% for lung cancer, 12-29% for kidney cancer, 25% for bladder cancer, 6-23% for ovarian cancer, 14-20% for head and neck cancer, 22% for gastric cancer, 24% for colorectal cancer, 18% for triple-negative breast cancer, 24% for mesothelioma and 87% for Hodgkin's lymphoma. Lejeune (2015) Melanoma Res 25: 373-375. For a more recent update on response rates for Nivolumab, Pembrolizumab, Atezolizumab and Durvalumab, see Balar and Weber (2017) and Iwai et al. (2017).

As the above-cited data show, the anti-PD-1/PD-L1 therapies are not producing impressive objective responses in the majority of patients. A number of combination therapies have been proposed by combining an immunomodulatory (e.g. an activator of costimulatory molecule or an inhibitor of immune checkpoint molecule) with a second agent such as an IAP inhibitor, a TOR kinase inhibitor, a HDM2 ligase inhibitor, a PIM kinase inhibitor, a HER3 kinase inhibitor, a Histone Deacetylase (HDAC) inhibitor, a Janus kinase inhibitor, an FGF receptor inhibitor, an EGF receptor inhibitor, a c-MET inhibitor, an ALK inhibitor, a CDK4/6-inhibitor, a PI3K inhibitor, a BRAF inhibitor, a CAR T cell (e.g., a CAR T cell targeting CD19), a MEK inhibitor, or a BCR-ABL inhibitor (WO 2016/054555). Recent reports have shown that IAP inhibitors enhance the effects of immune-checkpoint inhibitor anti-PD-1 in immunocompetent mouse syngeneic cancer models indicating that they are good candidates for combination with immunotherapy for the treatment of cancer. Chesi et al. (2016); Pinzon-Ortiz et al. (2016); Beug et al. (2017) Nat Commun. Feb 15; 8. doi: 10.1038/ncomms14278.

Similarly, treatment of cancer by the administration of an IAP antagonist has been proposed but administration of such IAP antagonist alone appears to be insufficient to treat certain cancers. The principle of combinations of a SMAC mimetic compound with an immunostimulatory or immunomodulatory agent has been proposed with the aim of enhancing the efficacy of SMAC mimetics in the treatment of cancer (WO 2017/143449). Clinical Trials involving Debio 1143 in combination with avelumab (ClinicalTrials.gov Identifier: NCT03270176), Birinapant in combination with pembrolizumab (ClinicalTrials.gov Identifier: NCT02587962), and LCL-161 in combination with PDR001 (ClinicalTrials.gov Identifier: NCT02890069) are currently underway. Further, Bo (2017) "Role of Smac in Lung Carcinogenesis and Therapy" doi: 10.1371/journal.pone.0107165 discloses the simultaneous administration of Debio1143 and an anti-PD-1 antibody. However, none of the treatment methods provided in the prior art disclose the use of an induction therapy as described herein.

There is still a need to improve combination therapies in order to enhance efficacy of cancer treatment or to allow some cancer patients to be eligible to such cancer treatment.

### SUMMARY OF THE INVENTION

Any references in the description to methods of treatment refer to the compounds, pharmaceutical compositions and medicaments of the present invention for use in a method for treatment of the human (or animal) body by therapy (or for diagnosis)

The present inventors propose that a patient having a tumor can be pretreated with an IAP antagonist, such as a SMAC mimetic to enhance the immunogenicity of the patient's tumor microenvironment. The pretreatment enhances the effectiveness of the treatment with an anti-PD-1 molecule to cause an immune response against the tumor.

Pretreatment with an IAP antagonist is expected to have several distinct advantages over simultaneous administration with an anti-PD-1 molecule. The pretreatment alters the tumor microenvironment, rendering the tumor susceptible to an anti-PD-1 molecule before the anti-PD-1 molecule is even first administered. This may increase the efficacy of the anti-PD-1 molecule treatment when compared with a concurrent treatment with IAP antagonist and an anti-PD-1 molecule. Pretreatment may also reduce the time needed to observe an anti-PD-1 molecule treatment-related response. Because the effectivity of the anti-PD-1 molecule may be increased by the pre-treatment, the patient may only need to be administered with less anti-PD-1 molecule over a shorter period of time.

Thus, the present disclosure relates to an induction therapy consisting of (the use of) an IAP antagonist for pretreating a subject diagnosed with a cancer to enhance the likelihood that a subsequent treatment with an anti-PD-1 molecule results in an anti-cancer response. In addition, or in the alternative, the use of the IAP antagonist, i.e., the induction therapy, is intended to enhance the responsiveness of the subject's cancer to the subsequent treatment with the anti-PD-1 molecule. While Applicant does not wish to be bound by any theory, it is likely that the enhancing effect of the IAP antagonist is due to an ability of the molecule to increase the immunogenicity of the subject's tumor microenvironment.

Thus, in one aspect, the present invention provides an inhibitor of apoptosis protein (IAP) antagonist for use in a method of treating cancer in a human subject, the method comprising:
(i) administering the IAP antagonist during an induction period, wherein the duration of the induction period is selected from the range of 1 to 48 days before first administration of an anti-PD-1 molecule; followed by
(ii) administering an anti-PD-1 molecule after the end of the induction period;
wherein:
the anti-PD-1 molecule is Nivolumab, Pembrolizumab, Atezolizumab, Durvalumab, Avelumab, PDR001, IBI-308, Cemiplimab, Camrelizumab, BGB-A317, BCD-100, JS-001, JNJ-3283, MEDI0680, AGEN-2034, TSR-042, Sym-021, PF-06801591, MGD-013, MGA-012, LZM-009, GLS-010, Genolimzumab, B! 754091, AK-104, CX-072, WBP3155, SHR-1316, PD-L1 Inhibitor millamolecule, BMS-936559, M-7824, LY-3300054, KN-035, FAZ-053, CK-301, or CA-170; and
the IAP antagonist administered during the induction period is Debio 1143, GDC-917/CUDC-427, LCL161, GDC-0152, TL-32711/Birinapant, HGS-1029/AEG-40826, B! 891065, ASTX-660 or APG-1387, preferably, the IAP antagonist is Debio 1143, LCL161 or Birinapant.

In a particular embodiment, the subject that is afflicted with a cancer is pretreated with the IAP antagonist during an induction or pretreatment period of 1 to 28 days, preferably 5 to 28 days, followed by the initiation of the subsequent anti-PD-1 molecule treatment. Of course, this means that no anti-PD-1 molecule is administered during the induction period. The induction period may include one or more days without administration of the IAP antagonist (days off). For example, there may be one or more days off between the last administration of the IAP antagonist during the induction period and the first administration of the anti-PD-1 molecule. If an IAP antagonist is used, which is administered daily, the induction period may include one or more days without the administration of the IAP antagonist.

In principle, any IAP antagonist can be used in the induction therapy. Preferably, the IAP antagonist is Debio 1143.

In the induction period, various doses and schedules are used for the selected IAP antagonist. The dose and schedule chosen may be dependent on various factors, such as the cancer type, the patient's characteristics and other therapies which the subject may be undergoing, and may be subject to the clinician's assessment and experience. For example, oral doses of between 500 and 1800 mg once weekly may be used for LCL-161, including 500 mg per os once weekly, 1200 mg per os once weekly, 1500 mg per os once weekly, 1800 mg per os once weekly. Birinapant may be used at doses between 13 and 47 mg/m², e.g. 47 mg/m² on days 1, 8 and 15 of 28-day cycles (days 2-7, 9-14 and 16-28 being days off Birinapant) or 13 mg/m² twice weekly for 3 weeks out of 4. Debio 1143 is administered orally in a daily amount of about 100 to about 1000 mg, preferably about 100 to about 500 mg, most preferably about 100 to about 250 mg, either every day during a period up to 28 days or in cycles comprising between 5 and 14 consecutive days of administration followed by 16 to 5 days off Debio 1143, such as 5 consecutive days of administration every 21 days, 14 consecutive days of administration every 21 days or 7 to 10 consecutive days of administration every 14 days.

In another embodiment, the cancer patient is not only administered the IAP antagonist prior to but also concurrently with the anti-PD-1 molecule treatment. The IAP antagonist treatment can be continued during the entire period during which the anti-PD-1 molecule is administered. Alternatively, co-administration of the IAP antagonist can be ended prior to the completion of the anti-PD-1 molecule treatment, or administration of the IAP antagonist can be continued beyond the completion of the anti-PD-1 molecule treatment.

The induction therapy, i.e. the use of an IAP antagonist for pretreating a cancer patient prior to treatment with an anti-PD-1 molecule, is not limited by the type of cancer the patient is afflicted with. In a particular embodiment, the cancer is of a type that is known to be responsive to treatment with an anti-PD-1 molecule in a substantial fraction of treated patients. This includes but is not limited to the types of cancers the anti-PD-1 molecule selected for treatment is licensed or recommended for. In a specific embodiment thereof, the cancer is head & neck cancer, melanoma, urothelial cancer, non-small cell lung cancer, microsatellite instability (MSI) high tumors from agnostic primary site or kidney cancer. In some embodiments, the cancer is a cancer for which the fraction of responders to treatment with an anti-PD-1 molecule is 10% or more, preferably 20% or more and more preferably 30% or more. In another embodiment, the cancer is of a type for which a low percentage of patients (e.g. 5% or less) have been shown to respond to treatment with an anti-PD-1 molecule and for which induction therapy according to the present invention would improve the response rate. This includes but is not limited to the types of cancers the anti-PD-1 molecule selected for treatment is not (yet) licensed or recommended for. In a specific embodiment thereof, the cancer is pancreatic cancer, colorectal cancer, multiple myeloma, small cell lung cancer, hepatocarcinoma or ovarian cancer.

In principle, any IAP antagonist can be used. Preferably, the IAP antagonist is Debio 1143.

In cases where the IAP antagonist is continued during anti-PD-1 molecule treatment, the same or a different IAP antagonist may be used as in the induction period, preferably the same. Preferably, the IAP antagonist is Debio 1143. Doses and schedules (cycles) may also be the same or different as in the induction period, as per the clinician's assessment and experience. Cycles may be repeated as long as there is observed clinical benefit either by no symptoms worsening, absence of disease progression as objectively evaluated by RECIST/iRECIST guidelines, and in the absence of unacceptable toxicity or until there is clinical need to change the therapeutic approach.

In principle, the anti-PD-1 molecule administered after the induction period can be any anti-PD-1 molecule. Specific anti-PD-1 molecules include Nivolumab, Pembrolizumab, Atezolizumab, Durvalumab, Avelumab, PDR001, IBI-308, Cemiplimab, Camrelizumab, BGB-A317, BCD-100, JS-001, JNJ-3283, MEDI0680, AGEN-2034, TSR-042, Sym-021, PF-06801591, MGD-013, MGA-012, LZM-009, GLS-010, Genolimzumab, B! 754091, AK-104, CX-072, WBP3155, SHR-1316, PD-L1 Inhibitor millamolecule, BMS-936559, M-7824, LY-3300054, KN-035, FAZ-053, CK-301 and CA-170. The anti-PD-1 molecule selected for treatment after the induction period is administered in an amount and at a dose schedule commonly used in clinical practice. In a particular embodiment, the anti-PD-1 molecule administered after the induction period may be combined with one or more other cancer therapies, including but not limited to other immunotherapies (such as other immunecheckpoint inhibitors including but not limited to anti-CTLA4 antibodies, IDO inhibitors, cell therapy, cancer vaccine, other immunomodulators), radiotherapy, chemotherapy, chemioradiotherapies, oncolytic viruses, anti-angiogenic therapies (such as VEGFR inhibitors), and/or targeted cancer therapies.

In a particular embodiment, the induction therapy of the present invention is made conditional on or is only recommended after an assessment that the cancer microenvironment is poorly immunogenic. Hence, in some embodiments, the patient is considered eligible for induction therapy after its cancer has been assessed to be poorly immunogenic. In some embodiments, the cancer has been assessed to be poorly immunogenic. For instance, in some embodiments, the cancer may have been assessed to be of low immunogenicity in accordance with one of the definitions provided herein below. The assessment typically involves an analysis of a marker of immunogenicity in a patient's biological sample such as a cancer biopsy (including liquid biopsy) taken prior to a pretreatment with an IAP antagonist and a finding that the presence, expression level or derived score of the marker does not attain a predetermined threshold. A preferred marker is PD-L1 expressed on cancer cells and/or immune cells. Other preferred markers include tumor-infiltrating lymphocytes and/or tumor mutation burden.

In another particular embodiment, treatment of a patient with an anti-PD-1 molecule is made conditional on or is only recommended after an assessment that the cancer is immunogenic at the end of the induction period, i.e., pretreatment with an IAP antagonist. In some embodiments, the cancer at the end of the induction period may have been assessed to be of high immunogenicity in accordance with one of the definitions provided herein below. The assessment typically involves an analysis of a marker of immunogenicity in a patient's biological sample such as a cancer biopsy (including liquid biopsy) taken after pretreatment of the patient with an IAP antagonist and a finding that the presence, expression level or derived score of the marker exceeds a predetermined threshold. A preferred marker is PD-L1 expressed on cancer cells and/or immune cells. Other preferred markers include tumor-infiltrating lymphocytes and/or tumor mutation burden.

In yet another particular embodiment, during the induction treatment, one or more other cancer therapies may be used, such as radiotherapy, chemotherapy, oncolytic viruses, targeted cancer therapies, cancer vaccine, cell therapy, and/or anti-angiogenic therapies. Any cancer co-therapy can be used during the induction period except an anti-PD-1 molecule therapy. Thus, an anti-PD-1 molecule is not administered during the induction period.

In a preferred embodiment of the present invention, Debio 1143 is used in the induction period (or as a pretreatment) as well as during the subsequent anti-PD-1 molecule treatment. During said subsequent treatment, the preferred anti-PD-1 molecule is Nivolumab, Pembrolizumab, Atezolizumab, Durvalumab, Avelumab, PDR 001 or Bl-754091. In a particularly preferred embodiment of the present invention, Debio 1143 is used for the treatment of head & neck cancer, melanoma, urothelial cancer, non-small cell lung cancer, microsatellite instability (MSI) high tumors from agnostic primary site, kidney cancer, pancreas cancer, colorectal cancer, multiple myeloma, small cell lung cancer, hepatocarcinoma or ovarian cancer in an induction period (or as a pretreatment) for a duration of 5 to 28 days as well as during the subsequent anti-PD-1 molecule treatment. During said subsequent treatment, the preferred anti-PD-1 molecule is Nivolumab, Pembrolizumab, Atezolizumab, Durvalumab, Avelumab, PDR 001 or BI-754091.

### BRIEF DESCRIPTION OF FIGURES

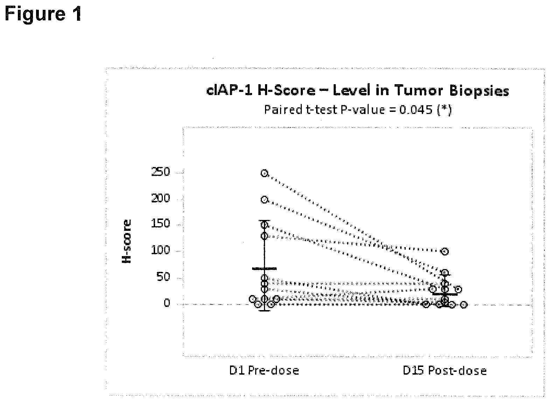

Figure 1 is a graph showing that Debio 1143 treatment induces the degradation of clAP1 in tumors of human head & neck cancer patients (n=12 patients), as per Example 1. Statistical analysis used a paired t-test and P-value = 0.045.

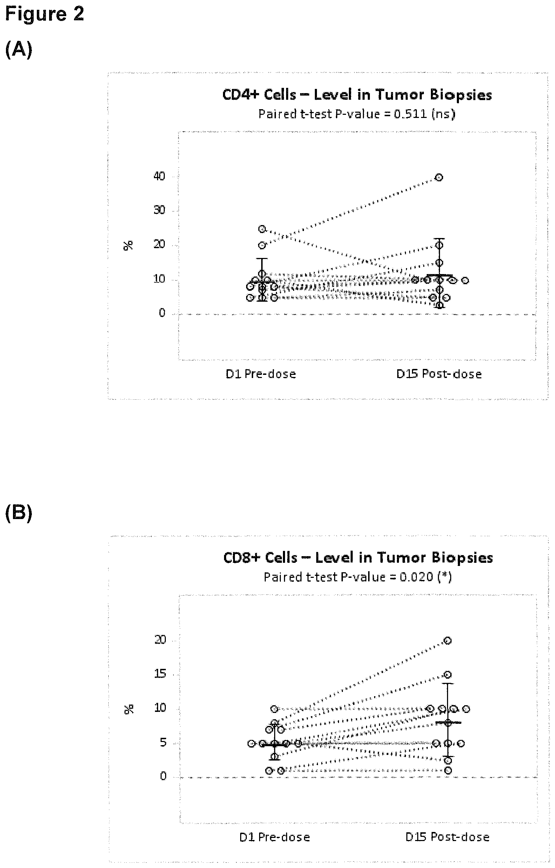

Figure 2 is a graph showing that Debio 1143 treatment increases the number of CD4+ (A) and CD8+ (B) T-lymphocytes in the tumor of head & neck cancer patients (n=12 patients), as per Example 1. Statistical analysis used a paired t-test. P-value for Figure 2(A) = 0.511 and P-value for Figure 2(B) = 0.020.

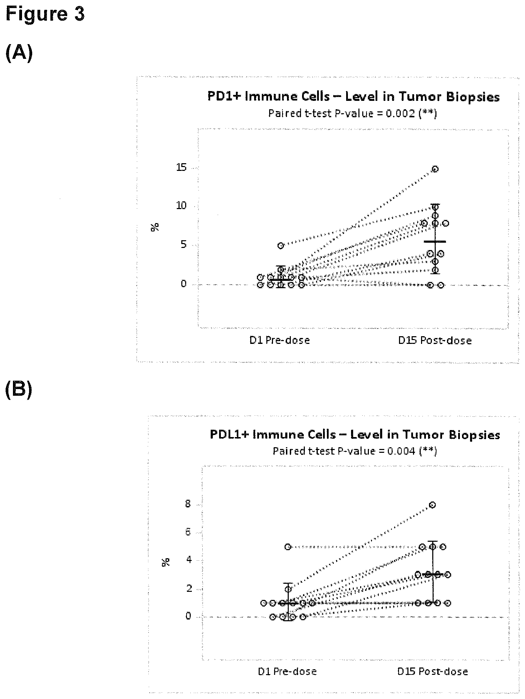

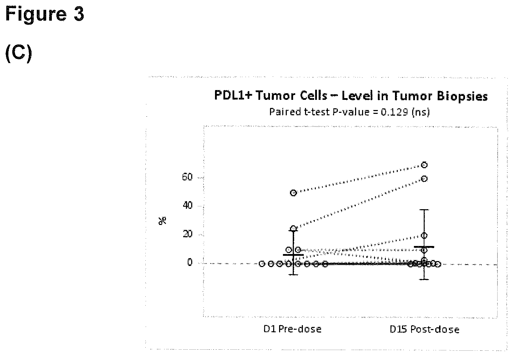

Figure 3 is a graph showing that Debio 1143 increases the number of PD-1+ immune cells (A) and PD-L1+ immune (B) and tumor (C) cells in the tumor of head & neck cancer patients (n=12 patients), as per Example 1. Statistical analysis used a paired t-test. P-value for Figure 3(A) = 0.002, P-value for Figure 3(B) = 0.004 and P-value for Figure 3(C) = 0.129.

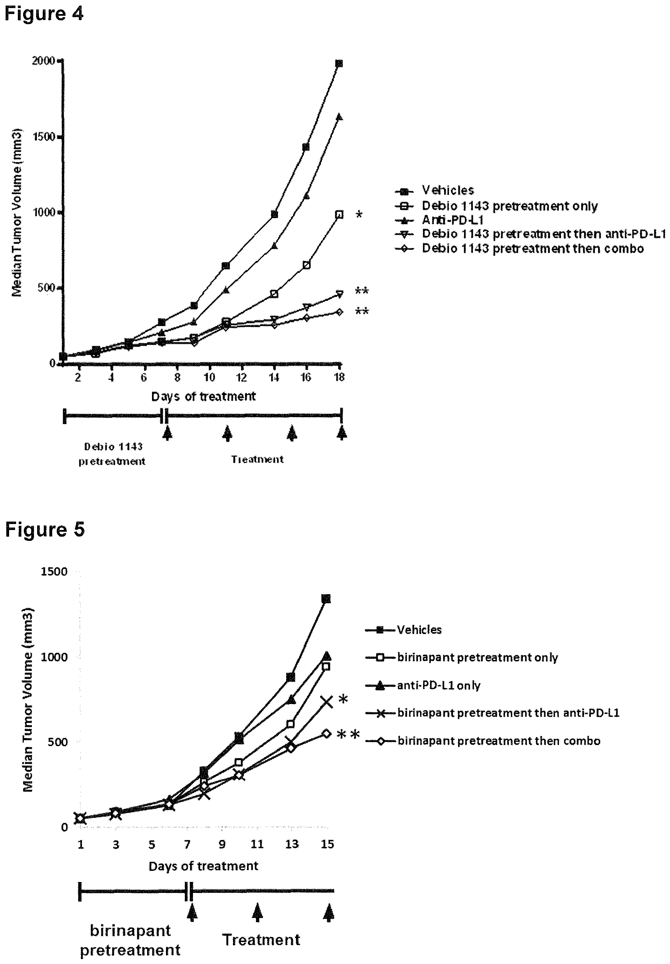

Figure 4 is a graph showing that pretreatment with Debio 1143 sensitizes MC38 tumors to a subsequent treatment with an anti-PD-L1 antibody, as measured by median tumor volume. At day of optimal T/C (day 18): p<0.05 (*) for Debio 1143 pretreatment only versus vehicles; p<0.0001 (**) for Debio 1143 pretreatment then PD-L1 versus vehicles; p<0.0001 (**) for Debio 1143 pretreatment then combo versus vehicles; as determined by student t-test (two-tailed, unpaired, equal variance). N=8 mice per group, except n=6 for vehicles on day 18. Note: combo= Debio 1143 + anti-PD-L1.
Figure 5 is a graph showing that pretreatment with birinapant sensitizes MC38 tumors to a subsequent treatment with an anti-PD-L1 antibody, as measured by median tumor volume. At day of optimal T/C (day 15): p>0.05 for birinapant pretreatment only versus vehicles; p<0.05 (*) for birinapant pretreatment then PD-L1 versus vehicles; p<0.001 (**) for birinapant pretreatment then combo versus vehicles; as determined by student t-test (two-tailed, unpaired, equal variance). N=8 mice per group. Note: combo= birinapant + anti-PD-L1.

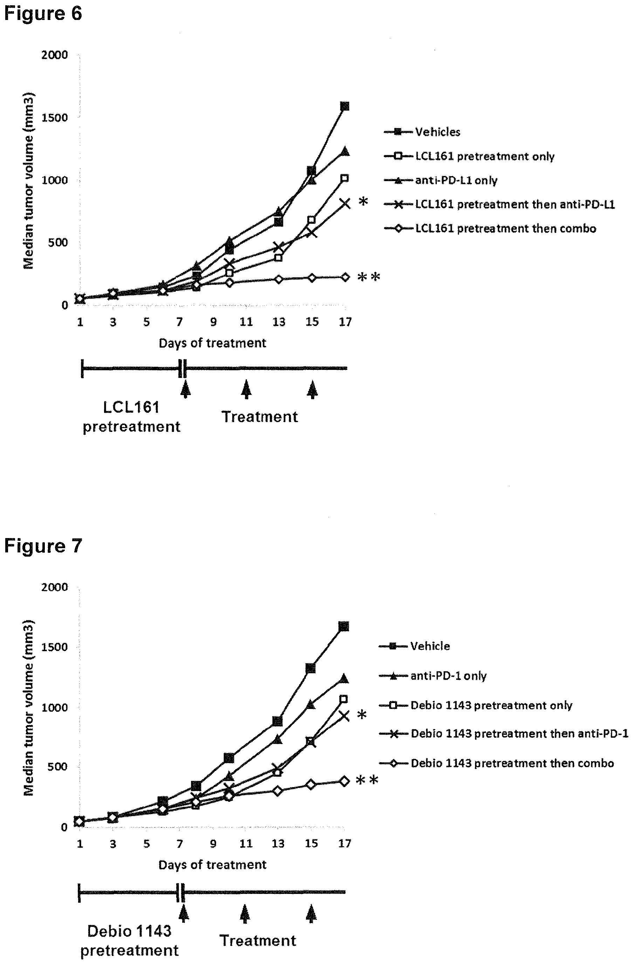

Figure 6 is a graph showing that pretreatment with LCL161 sensitizes MC38 tumors to a subsequent treatment with an anti-PD-L1 antibody, as measured by median tumor volume. At day of optimal T/C (day 15): p<0.05 (*) for LCL161 pretreatment only versus vehicles; p<0.05 (*) for LCL161 pretreatment then PD-L1 versus vehicles; p<0.001 (**) for LCL161 pretreatment then combo versus vehicles; as determined by student t-test (two-tailed, unpaired, equal variance). N=8 mice per group. Note: combo= LCL161 + anti-PD-L1.
Figure 7 is a graph showing that pretreatment with Debio 1143 sensitizes CT26 tumors to a subsequent treatment with an anti-PD-1 antibody, as measured by median tumor volume. At day of optimal T/C (day 17): p>0.05 for Debio 1143 pretreatment only versus vehicles; p<0.05 (*) for Debio 1143 pretreatment then PD-1 versus vehicles; p<0.0001 (**) for Debio 1143 pretreatment then combo versus vehicles; as determined by student t-test (two-tailed, unpaired, equal variance). N=8 mice per group, except n=7 for vehicles on day 17. Note: combo= Debio 1143 + anti-PD-1.

### DETAILED DESCRIPTION OF THE INVENTION

### Definitions

The terms "antagonist" and "inhibitor" are used interchangeably and refers to a substance which interferes with or inhibits the physiological action of another. In some embodiments, the terms "antagonist" and "inhibitor" have the same meaning as understood by the person skilled in the art at the first priority date, i.e. December 21, 2017, bearing in mind the skilled person's common general knowledge at the first priority date.

The term "antibody" refers to a molecule comprising at least one immunoglobulin domain that binds to, or is immunologically reactive with, a particular antigen. The term includes whole antibodies and any antigen binding portion or single chains thereof and combinations thereof. The term "antibody" in particular includes bispecific antibodies.

A typical type of antibody comprises at least two heavy chains ("HC") and two light chains ("LC") interconnected by disulfide bonds.

Each "heavy chain" comprises a "heavy chain variable domain" (abbreviated herein as "VH") and a "heavy chain constant domain" (abbreviated herein as "CH"). The heavy chain constant domain typically comprises three constants domains, CH1, CH2, and CH3.

Each "light chain" comprises a "light chain variable domain" (abbreviated herein as "VL") and a "light chain constant domain" ("CL"). The light chain constant domain (CL) can be of the kappa type or of the lambda type. The VH and VL domains can be further subdivided into regions of hypervariability, termed Complementarity Determining Regions ("CDR"), interspersed with regions that are more conserved, termed "framework regions" ("FW").

Each VH and VL is composed of three CDRs and four FWs, arranged from aminoterminus to carboxy-terminus in the following order: FW1, CDR1, FW2, CDR2, FW3, CDR3, FW4. The present disclosure inter alia presents VH and VL sequences as well as the subsequences corresponding to CDR1, CDR2, and CDR3.

Accordingly, a person skilled in the art would understand that the sequences of FW1, FW2, FW3 and FW4 are equally disclosed. For a particular VH, FW1 is the subsequence between the N-terminus of the VH and the N-terminus of H-CDR1, FW2 is the subsequence between the C-terminus of H-CDR1 and the N-terminus of H-CDR2, FW3 is the subsequence between the C-terminus of H-CDR2 and the N-terminus of H-CDR3, and FW4 is the subsequence between the C-terminus of H-CDR3 and the C-terminus of the VH. Similarly, for a particular VL, FW1 is the subsequence between the N-terminus of the VL and the N-terminus of L-CDR1, FW2 is the subsequence between the C-terminus of L-CDR1 and the N-terminus of L-CDR2. FW3 is the subsequence between the C-terminus of L-CDR2 and the N-terminus of L-CDR3, and FW4 is the subsequence between the C-terminus of L-CDR3 and the C-terminus of the VL.

The variable domains of the heavy and light chains contain a region that interacts with an antigen, and this region interacting with an antigen is also referred to as an "antigenbinding site" or "antigen binding site" herein. The constant domains of the antibodies can mediate the binding of the immunoglobulin to host tissues or factors, including various cells of the immune system (e.g., effector cells) and the first component (C1q) of the classical complement system. Exemplary antibodies of the present disclosure include typical antibodies, but also fragments and variations thereof such as scFvs, and combinations thereof where, for example, an scFv is covalently linked (for example, via peptidic bonds or via a chemical linker) to the N-terminus of either the heavy chain and/or the light chain of a typical antibody, or intercalated in the heavy chain and/or the light chain of a typical antibody. Further, exemplary antibodies of the present disclosure include bispecific antibodies.

As used herein, the term "antibody" encompasses intact polyclonal antibodies, intact monoclonal antibodies, antibody fragments (such as Fab, Fab', F(ab')2, and Fv fragments), single chain variable fragment (scFv), disulfide stabilized scFvs, multispecific antibodies such as bispecific antibodies, chimeric antibodies, humanized antibodies, human antibodies, fusion proteins comprising an antigen determination portion of an antibody, and any other modified immunoglobulin molecule comprising an antigen binding site.

An antibody can be of any the five major classes (isotypes) of immunoglobulins: IgA, IgD, IgE, IgG, and IgM, or subclasses thereof (e.g. IgG1, IgG2, IgG3, IgG4, lgA1 and IgA2), based on the identity of their heavy-chain constant domains referred to as alpha, delta, epsilon, gamma, and mu, respectively. The different classes of immunoglobulins have different and well known subunit structures and three-dimensional configurations. Antibodies can be naked or conjugated to other molecules such as therapeutic agents or diagnostic agents to form immunoconjugates. In some embodiments, the term "antibody" has the same meaning as understood by the person skilled in the art at the first priority date, i.e. December 21, 2017, bearing in mind the skilled person's common general knowledge at the first priority date.

The terms "anti-cancer response", "response" or "responsiveness" relate to objective radiological and clinical improvements assessed using RECIST v1.1 criteria (Eur. J. Cancer 45; 2009: 228-247). RECIST is a set of published rules that define objectively when cancer patients improve ("respond"), stay the same ("stable") or worsen ("progression") during treatments. RECIST 1.1 has recently been adapted for evaluation of immunotherapeutic agents iRECIST 1.1. (Seymour, L., et al., iRECIST: Guidelines for response criteria for use in trials testing immunotherapeutics. Lancet Oncol, 2017. 18(3): p. e143-e152). In the present invention, a patient is considered to respond to a given treatment if there is any clinical benefit for the patient as per RECIST v 1.1, assessed as complete response (CR), partial response (PR) or stable disease (SD) or as having an increased duration of the response or disease stabilization as measured by progression free survival or overall survival status.

The term "anti-PD-1 molecule" refers to PD-1 inhibitors and PD-L1 inhibitors. These inhibitors include but are not limited to antibodies targeting PD-1 or PD-L1. The anti-PD-1 molecule may be a small molecule such as CA-170 (AUPM-170, Curis, Aurigene, described e.g. in J.J. Lee et al., Journal of Clinical Oncology 35, no. 15_suppl, DOI: 10.1200/JCO.2017.35.15_suppl.TPS3099). Further small molecule inhibitors of the PD-1/PD-L1 interaction are described in WO 2018/195321 A.

"Cancer" generally refers to malignant neoplasm, which may be metastatic or nonmetastatic. For instance, non-limiting examples of cancer that develops from epithelial tissues such as gastrointestinal tract and skin include non-melanoma skin cancer, head and neck cancer, esophageal cancer, lung cancer, stomach cancer, duodenal cancer, breast cancer, prostate cancer, cervical cancer, cancer of endometrial uterine body, pancreatic cancer, liver cancer, cholangiocarcinoma, gallbladder cancer, colorectal cancer, colon cancer, bladder cancer, and ovarian cancer. Non-limiting examples of sarcoma that develops from non-epithelial tissues having mesodermal origin (stroma) such as muscles include osteosarcoma, chondrosarcoma, rhabdomyosarcoma, leiomyosarcoma, liposarcoma, gastrointestinal stromal tumors (GIST) and angiosarcoma. Non-limiting examples of tumors from an ectodermal (neural crest ontogeny) include brain tumors, neuroendocrine tumors, etc. Furthermore, non-limiting examples of hematological cancer derived from hematopoietic organs include malignant lymphoma including Hodgkin's lymphoma and non-Hodgkin's lymphoma, leukemia including acute myelocytic leukemia, chronic myelocytic leukemia, acute lymphatic leukemia, chronic lymphatic leukemia, and multiple myeloma. The latter examples of cancer are also referred to herein as types of cancer.

The terms "cancer" and "tumor" (meaning malignant tumor) are used interchangeably herein.

The term "concurrent therapy", "concurrent treatment" or "co-therapy" refers to the contemporaneous or simultaneous administration of both the IAP antagonist and the anti-PD-1 molecule. In some embodiment, the term "concurrent therapy" or "concurrent treatment" refers to a treatment wherein the IAP antagonist is not given sufficient time to enhance the immunogenic potency of a tumor's microenvironment before the anti-PD-1 molecule is administered. In some embodiments, the terms "concurrent treatment", "co-therapy" and "concurrent therapy" has the same meaning as understood by the person skilled in the art at the first priority date, i.e. December 21, 2017, bearing in mind the skilled person's common general knowledge at the first priority date.

"Effective amount" of an IAP antagonist or an anti-PD-1 molecule means the amount of compound that will elicit the biological or medical anti-cancer response sought by the clinician.

The phrase "to enhance the immunogenic potency of a tumor's microenvironment" refers to a stimulation of the immune system in the tumor microenvironment which results in an increased immune response in comparison to an unstimulated immune system. In the present case, the immune system may be stimulated by an IAP antagonist. The stimulation may increase the immunogenicity of the cancer, the stimulation may increase the amount of effector cells at the tumor microenvironment, and/or the stimulation may increase the sensitivity of immune effector cells present in the tumor microenvironment towards the cancerous cells. In some embodiments, the phrase "to enhance the immunogenic potency of a tumor's microenvironment" has the same meaning as understood by the person skilled in the art at the first priority date, i.e. December 21, 2017, bearing in mind the skilled person's common general knowledge at the first priority date.

The term "first administration" of an anti-PD-1 molecule, as used herein, specifies that the anti-PD-1 molecule is administered for the first time to a patient. In some embodiments, the patient has never been previously treated with an anti-PD-1 molecule. In some embodiments, the patient has been treated with an anti-PD-1 molecule but the patient has relapsed or the anti-PD-1 molecule therapy was ineffective. In these embodiments, the previously administered anti-PD-1 molecule level in the serum has been sufficiently reduced, e.g. by 95%, before the induction therapy of the present invention is started. In some embodiments, the time between the last administration of the previously administered anti-PD-1 molecule and the start of the induction therapy of the present invention represents at least one or two dosing interval (time between repeated administration) as approved by regulatory agencies or accepted by the medical community. In some embodiments, the subject has not been administered with an anti-PD-1 molecule for at least, 1, 2, 3, 4 or even 6 weeks before the start of the induction period.

The terms "immunogenic" and "immunogenicity" as used herein in relation to the tumor microenvironment means causing or producing an immune response. In some embodiments, immunogenicity is assessed by determining the expression level of PD-L1 revealed by immunostaining on the patient's cancer cells.

In some embodiments, immunogenicity is assessed by considering the level of CD8+ cells in the cancer sample as a marker. This assessment may be carried out using the materials and methods of Example 1 below.

In some embodiments, cancer samples may be assessed and classified as being of low and high immunogenicity by considering the above-mentioned markers in combination. Hence, in some embodiments, immunogenicity is assessed by considering a combination of the PD-L1 marker expression levels together with the level of CD8+ cells in the cancer sample. If, in some embodiments, the treatment with IAP antagonist during the induction period increases the expression level of PD-L1 on the patient's cancer cells, for example by at least 1, 2, 3 or 4 % in terms of the fraction of cells of a cancer sample exhibiting staining for PD-L1 (at any intensity) in an immunohistochemistry assay using a suitable antibody such as, for example, antibody 22c3 pharmDx (Dako, Inc.), the treatment is with IAP antagonist is considered to enhance the immunogenic potency of the tumor's microenvironment. Similarly, an enhancement in immunogenic potency may be identified in some embodiments by means of an increase in the level of CD8+ cells in the cancer sample by at least 1, 2, 3 or 4 %, when determined using the materials and methods of Example 1 below.

IAP antagonist or inhibitor as used herein means a compound having affinity for inhibitor of apoptosis proteins (abbreviated as IAP). The compound is an inhibitor or antagonist of lAPs. In some embodiments, the IAP antagonist shows the characteristic that an interaction between the IAP antagonist and clAP1 and/or clAP2 leads to degradation of these proteins and subsequent NF-κB modulation. In some embodiments, this effect can be used for testing a compound for IAP inhibitory activity: when contacting the potential IAP antagonist with clAP1 and/or clAP2 in vitro and analyzing the effect with a suitable technique including but not limited to western blot analysis, for an IAP inhibitor, an effect on clAP1 should be observed at concentrations below 10 µM, preferably, <1 µM. In some embodiments, the term "IAP inhibitor" and "IAP antagonist" has the same meaning as understood by the person skilled in the art at the first priority date, i.e. December 21, 2017, bearing in mind the skilled person's common general knowledge at the first priority date.

In general, the term "induction therapy" refers to a type of treatment wherein a drug is administered to a patient to induce a response in the patient that potentiates the effectiveness of another drug that is administered afterwards. In the context of the present invention, the induction therapy involves a "pretreatment". The "pretreatment" or "induction" refers to the administration of an IAP antagonist for a certain amount of time before the first administration of the anti-PD-1 molecule. The period in which the IAP antagonist is administered is referred to as the "induction period" or "pretreatment period". The induction period is not particularly limited as long as the immunogenic potency of a tumor's microenvironment is enhanced. In the invention, the induction period has a duration selected from the range of 1 to 48 days, preferably 1 to 28 days, more preferably 5 to 28 days. In some embodiments, the induction period is sufficiently long to enhance the immunogenic potency of a tumor's microenvironment. In some embodiments, the efficacy of the anti-PD-1 molecule treatment is increased in comparison with a concurrent treatment without induction therapy with an IAP antagonist. The anti-PD-1 molecule is then administered after the induction period, i.e. after the immunogenic potency of a tumor's microenvironment has been enhanced. This results in an increased potency of the anti-PD-1 molecule because the immune system has been primed by the IAP antagonist. In some embodiments, the terms "induction therapy", "pretreatment", "induction", "induction period" and "pretreatment period" have the same meaning as understood by the person skilled in the art at the first priority date, i.e. December 21, 2017, bearing in mind the skilled person's common general knowledge at the first priority date.

"SMAC mimetic" means a small-molecule inhibitor for therapeutic inhibition of IAP which small-molecule inhibitor mimics the N-terminal four-amino acid stretch of the endogenous SMAC sequence and is at least partly comprised of non-peptidic elements. The N-terminal sequence of endogenous SMAC is Ala-Val-Pro-Ile (AVPI) and is required for binding to IAP.

The term "subject" relates to a mammalian animal and, preferably, to a human person. A human subject is also referred to as a "patient".

### Induction therapy

Inventors propose that a patient having a tumor can be pretreated with an IAP antagonist, such as a SMAC mimetic to enhance the immunogenicity of the patient's tumor microenvironment. Subsequently, the patient is treated with an anti-PD-1 molecule. The pretreatment increases the likelihood that a patient's tumor will respond to a treatment with an anti-PD-1 molecule and/or enhances the effectiveness of the tumor's response to an anti-PD-1 molecule. The IAP antagonist may be selected among those that are already (as at December 21, 2017) approved or are currently in clinical development, in particular among the following ones:
Debio 1143 (Debiopharm, CAS RN: 1071992-99-8), GDC-917/CUDC-427 (Curis/Genentech, CAS RN: 1446182-94-0), LCL161 (Novartis, CAS
RN: 1005342-46-0), GDC-0152 (Genentech, CAS RN: 873652-48-3),
TL-32711/Birinapant (Medivir, CAS RN: 1260251-31-7), HGS-1029/AEG-408268 (Aegera, CAS RN: 1107664-44-7), BI 891065 (Boehringer Ingelheim), ASTX-660 (Astex/Otsuka, CAS RN: 1605584-14-2), APG-1387 (Ascentage, CAS
RN: 1802293-83-9), or any of their pharmaceutical acceptable salts. Preferably, the IAP antagonist is Debio 1143.

Pretreatment with an IAP antagonist may be made dependent on a finding that the patient's tumor microenvironment is poorly immunogenic. Immunogenicity may be assessed in a patient's biological sample, such as a tumor biopsy (including liquid biopsy) taken prior to pretreatment. Criteria for immunogenicity that may be employed include the level of PD-L1 expressed in the cancerous cells or in all cells present in the cancer biopsy. It may also be the percentage of tumor cells and/or immune cells expressing detectable amounts of PD-L1. The threshold for immunogenicity may be defined by the medical community, the manufacturer/ distributor of the anti-PD-1 molecule to be used for analysis or the treating physician. For example, the threshold level for treatment with Pembrolizumab has been defined by the manufacturer (Merck) as more than 50% of cells of the cancer staining for PD-L1 (at any intensity) in an immunohistochemistry assay using antibody 22c3 pharmDx (Dako, Inc.) for first line therapy, and more than 1% of cells staining for PD-L1 for second line therapy. Hence, in this example, patients with cancers with lower frequencies of PD-L1-expressing cells would be considered eligible for pretreatment with an IAP antagonist. In some embodiments, pretreatment with an IAP antagonist may be carried out until the frequency of PD-L1-expressing cells and/or CD8+ cells exceeds the above-mentioned threshold levels for high immunogenicity. Additional criteria may include the percentage of lymphocytes, or CD8+ T cells, or CD4+ T cells present in the baseline biopsy or sample. Other suitable criteria of immunogenicity may gain acceptance by the medical community (e.g., number/percentage of dendritic cells, ratio of CD8+ T cells to regulatory T cells, tumor mutation burden, etc.). Eligibility may also be assessed based on multiple criteria.

Without being bound to a particular theory, an increase in the expression of the PD-L1 marker on cancer cells after the induction period is believed to be a sign that the immunogenic potency of the tumor microenvironment has been enhanced. This is because an increased immunogenic potency should be associated with an increased need to circumvent the immune system for the cancer cell to survive. Overexpression of PD-L1 is thought to be a mechanism with which the cancer cell can hide from the immune system. Thus, an increased level of PD-L1 expression is a sign that the tumor cell is being confronted with an enhanced immune system at the tumor microenvironment.

The method may also be adapted to select patients for treatment with an anti-PD-1 molecule based on the immunogenicity of their cancer microenvironment at the end of a pretreatment with an IAP antagonist. Immunogenicity may be assessed in a patient's biological sample, such as a tumor biopsy (including liquid biopsy) taken at the end of the pretreatment. Criteria for assessing immunogenicity and for defining thresholds may be similar to those that have been described in the previous section. Patients with cancers for which the selected marker of immunogenicity surpasses a predetermined threshold may be selected for treatment with an anti-PD-1 molecule.

### Methods for assessing immunogenicity in cancer biopsies

In principle, any suitable method may be employed. Most often used are procedures based on immunohistochemistry and flow cytometry.

Immunohistochemistry: Immunohistochemistry (IHC) is a method capable of demonstrating the presence and location of proteins in tissue sections. It enables the observation of processes in the context of intact tissue. The basic steps of the IHC protocol are as follows: fixing and embedding the tissue, cutting and mounting the section, deparaffinizing and rehydrating the section, applying antigen retrieval process, immunohistochemical staining and viewing the staining under the microscope. In an example protocol, immunostaining was performed on 4-µm paraffin-embedded tissue sections. Briefly, slides were deparaffinized in xylene and dehydrated utilizing a graded ethanol series, and endogenous peroxidase was blocked with 3% hydrogen peroxide. After epitope retrieval, the slides were washed with and blocked with TRIS-buffered saline with 0.1% (vol.) Tween 20/5% (vol.) normal goat serum. Incubation with a primary antibody was performed overnight at 4°C followed by incubation with a secondary antibody for 30 min at room temperature. Sections were washed three times with TRIS-buffered saline with 0.1% (vol.) Tween 20, stained with diaminobenzidine (DAB) and counterstained with hematoxylin. Guancial et al. (2014); Redler, A. et al. (2013) PLoS One. 8: e72224. Procedures may be carried out manually or may be partially or completely automated. A specific IHC method is also described in the example section below.

Flow Cytometry: Flow cytometry is a laser-based, biophysical technology employed in cell counting, cell sorting, biomarker detection and protein engineering, involving suspending cells in a stream of fluid and passing them by an electronic detection apparatus. It allows simultaneous multiparametric analysis of the physical and chemical characteristics of up to thousands of particles per second. Using antibody specific of protein, flow cytometry can provide information regarding the expression of cell surface and, in some cases, cytoplasmic or nuclear markers that are used to understand complex cellular populations or processes. Yan, D. et al. (2011) Arthritis Res.Ther. 13: R130.

### Pharmaceutical compositions comprising an IAP antagonist and their administration

Pharmaceutical compositions comprising an IAP antagonist may be administered orally, parenterally, by inhalation spray, topically, rectally, nasally, buccally, vaginally or via an implanted reservoir, preferably by oral administration or administration by injection. However, it is noted that dimeric SMAC mimetics are typically administered intravenously. The pharmaceutical compositions may contain any conventional non-toxic pharmaceutically acceptable carriers, adjuvants or vehicles. In some cases, the pH of the formulation may be adjusted with pharmaceutically acceptable acids, bases or buffers to enhance the stability of the active agent or its delivery form. Standard pharmaceutical carriers and their formulations are described, in a non-limiting fashion, in Remington's Pharmaceutical Sciences, Mack Publishing Co., Easton, PA, 19th ed. 1995. The term parenteral as used herein includes subcutaneous, intracutaneous, intravenous, intramuscular, intraarticular, intraarterial, intrasynovial, intrasternal, intrathecal, intralesional and intracranial injection or infusion techniques.

Liquid dosage forms for oral administration include pharmaceutically acceptable emulsions, microemulsions, solutions, suspensions, syrups and elixirs. In addition to active agent (IAP antagonist, such as a SMAC mimetic), the liquid dosage forms may contain inert diluents commonly used in the art such as, for example, water or other emulsifiers, solubilizing agents and solvents such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions, may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride and dextrose solutions. In addition, sterile, fixed oils are conventionally employed as a solvent or suspending medium. For this purpose, any bland fixed oil can be employed including synthetic mono- or diglycerides. In addition, fatty acids such as oleic acid are used in the preparation of injectables.

The injectable formulations can be sterilized, for example, by filtration through a bacteriaretaining filter, ionizing radiation, or by incorporating active agent in the form of a sterile solid composition which can be dissolved or dispersed in sterile water or other sterile injectable medium prior to use. Depending on the chemical nature of the particular IAP antagonist employed, sterilization may also be by autoclaving or dry heat.

In order to prolong the effect of the active agent, it is often desirable to slow the absorption of the active agent from subcutaneous or intramuscular injection. This may be accomplished by the use of a liquid suspension of crystalline or amorphous material with poor water solubility. The rate of absorption of the active agent then depends upon its rate of dissolution, which, in turn, may depend upon crystal size and crystalline form. Alternatively, delayed absorption of a parenterally administered drug form is accomplished by dissolving or suspending the active agent in an oil vehicle. Injectable depot forms are made by microencapsulating the active agent in biodegradable polymers such as polylactide-polyglycolide. Depending upon the ratio of active agent to polymer and the nature of the particular polymer employed, the rate of release of the active agent can be controlled. Examples of other biodegradable polymers include poly(orthoesters) and poly(anhydrides). Depot injectable formulations are also prepared by entrapping the active agent in liposomes or microemulsions that are compatible with body tissues.

Solid dosage forms for oral administration include capsules, tablets, pills, powders and granules. In such solid dosage forms, active agent is mixed with at least one inert, pharmaceutically acceptable excipient or carrier such as sodium citrate or dicalcium phosphate and/or: a) fillers or extenders such as starches, lactose, cellulose, sucrose, glucose, mannitol, and silicic acid, b) binders such as, for example, carboxymethylcellulose, alginates, gelatin, polyvinylpyrrolidone, sucrose, and acacia, c) humectants such as glycerol, d) disintegrating agents such as agar-agar, calcium carbonate, croscarmellose, crospovidone, carboxymethylcellulose, potato or tapioca starch, alginic acid, certain silicates, and sodium carbonate, e) solution-retarding agents such as paraffin, f) absorption accelerators such as quaternary ammonium compounds, g) wetting agents such as, for example, cetyl alcohol, sodium lauryl sulfate and glycerol monostearate, h) absorbents such as kaolin and bentonite clay, and/or i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active agent only, or preferentially, in a certain part of the intestinal tract, optionally, in a delayed manner. Examples of embedding compositions that can be used include polymeric substances and waxes.

The amount of active agent that may be combined with pharmaceutically acceptable excipients or carriers to produce a single dosage form will vary depending on the particular IAP antagonist chosen, the particular mode of administration and, possibly, the subject treated. A typical preparation will contain from 1% to 95% active agent (w/w). Alternatively, such preparations may contain from 20% to 80% active agent. Lower or higher doses than those recited above may be required. Specific dosage and treatment regimens for any particular subject will depend upon a variety of factors, including the age, body weight, body surface area, general health status, sex, diet, time of administration, rate of excretion, IAP antagonist, drug combination, the severity and course of the disease, condition or symptoms, the subject's disposition to the disease, condition or symptoms, and the judgment of the treating physician.

### Pharmaceutical compositions comprising an anti-PD-1 molecule and their administration

Anti-PD-1 molecules are administered typically by intravenous infusion.

Nivolumab is being distributed under the brand "OPDIVO". It comes as a 10 mg/ml solution that comprises the Nivolumab antibody, mannitol, pentetic acid, polysorbate 80, sodium chloride, sodium citrate dihydrate and water. For administration, it is diluted into 0.9% sodium chloride or 5% dextrose. Pembrolizumab is being distributed under the brand "KEYTRUDA". It is furnished as a solid composition comprising 50 mg antibody and inactive ingredients L-histidine, polysorbate-80 and sucrose. For administration, the composition is suspended in 0.9% sodium chloride. Atezolizumab (brand name: "TECENTRIQ") is provided as an IV solution (1200 mg active/20 ml) containing glacial acetic acid, histidine, sucrose and polysorbate 20. For administration, the solution is diluted with 0.9% NaCl. Durvalumab ("IMFINZI") comes as 500 mg/10 ml or 120 mg/2.4 ml solutions in L-histidine, L-histidine hydrochloride monohydrate, α,α-trehalose dihydrate, polysorbate 80, and water for injection, USP. Avelumab ("BAVENCIO") is marketed as a 200 mg (active)/10 ml solution for injection that contains mannitol, acetic acid, polysorbate 20, sodium hydroxide and water. After dilution in 0.45% or 0.9% NaCl, an appropriate dose is administered by infusion during 60 min.

Suitable doses of checkpoint inhibitors are those used in the clinic. A suitable dose of Nivolumab is 3 mg/kg body weight. This dose is administered by intravenous infusion during a period of 60 min. A suitable dose of Pembrolizumab is 2 mg/kg body weight. This dose is administered by intravenous infusion during a period of 30 min. The adult dose of Atezolizumab is 1200 mg infused over a period of 60 min. The recommended dose for Durvalumab is 10 mg/kg body weight administered by intravenous infusion over 60 min. A suitable dose for Avelumab is 10 mg/kg body weight. These doses may be adapted in parallel with adaptations accepted in clinical practice. Dosing of Nivolumab is typically repeated every two weeks, Pembrolizumab every three weeks, Atezolizumab every three weeks, Durvalumab every two weeks and Avelumab every two weeks.

Dose amounts and schedules (including dosing intervals) of administration of anti-PD-1 molecules will be as approved by regulatory agencies. Any modification of doses and schedules accepted by the medical community will also be applied to the presently described therapy.

### EXAMPLES

### Example 1: Pre-operative window-of-opportunity study of Debio 1143 with or without cisplatin (CDDP) in patients with resectable squamous cell carcinoma of the head and neck (EUDRACT 2014-004655-31)

For this clinical trial, Debio 1143 was used under its free base and formulated with starch and filed within hard gelatin capsules.

The main objective of this clinical trial was to investigate the pharmacodynamic activity of Debio 1143, alone or in combination with cisplatin, in patients with squamous cell carcinoma of the head and neck. Among the numerous secondary objectives, potential effects on immune signaling were also examined.

The study enrolled adult patients with newly diagnosed histologically proven squamous cell carcinoma of the oral cavity, oropharynx, hypopharynx or larynx. During a screening period of two weeks (days -14 to -1), a tumor biopsy was taken and analyzed. Treatment was from day 1 to day 15 (+/- 2 days) and consisted (in one arm) of daily administration p.o. of 200 mg Debio 1143. At the end of this treatment period, a second tumor biopsy was taken and analyzed, and the patients underwent surgery.

Biopsies were analyzed by immunohistochemical methods. Staining for clAP1 was carried out using a Dako autostainer automaton (Agilent). The EPR4673 mouse mAb (Abcam) was utilized at a 1/100 dilution, and tissue slides were exposed to the antibody for 20 min. Pretreatment of the slides was with EnVision FLEX Target Retrieval Solution, Low pH; the EnVision FLEX system (chromogen: DAB) was employed for visualization of the signal. EnVision Flex system and reagent were from Agilent. The same protocol was applied for PD-L1 staining. The E1L3N rabbit mAb (Cell Signaling Technology) was used at a 1/500 dilution.

T cells were identified using CD3 rabbit mAb 2GV6 from Ventana Roche (provided as a ready-to-use solution). Slides were processed on a Ventana Benchmark Ultra automaton. Exposure to antibody was 20 min. Pretreatment of the slides (64 min) was with cell conditioning solution CC1 (Ventana); the Optiview system (Ventana) (chromogen: DAB) was employed for visualization of the signal. Staining of CD8 and CD4 T cells was by the same protocol. The CD8 antibody was the SP57 rabbit mAb, and the CD4 antibody was the SP35 rabbit mAb. Both antibodies were from Ventana Roche and were provided as ready-to-use solutions. The antibody selected for PD-1 detection was the NAT105 mouse mAb that was also provided as a ready-to-use solution (Cell Marque). The protocol for PD-1 detection was the same as that used for CD3 staining, except that antibody exposure and pretreatment times were each 16 min.

Data obtained from 12 evaluable patients are discussed. As can be seen in Figure 1, treatment with Debio 1143 reduced levels of clAP1 in the tumors of most patients (p-value of 0.045 using paired t-test), demonstrating that an effective tumor concentration of the SMAC mimetic had been reached. The treatment also resulted in substantial increases in tumor-infiltrating lymphocytes as evidenced by the findings that numbers of CD4+ and CD8+ T cells in the tumor microenvironment were elevated as a consequence of the treatment (Figure 2). Statistical analysis of the data revealed that mean CD8+ and CD4+ T cell numbers were both increased, the increase in CD8+ T cell number being significant (p-value of 0.020 with paired t-test) (Figure 2(B)). The percentages of immune cells expressing PD-1 or PD-L1 increased significantly in treated tumors (Figure 3(A), p-value of 0.002 and (B), p-value of 0.004). In most tumors, the frequency of PD-L1-expressing cells was also increased (Figure 3(C)). Overall, the data strongly suggest that treatment with Debio 1143 enhances the immunogenicity of the tumor microenvironment in the human patients.

### Example 2: Animal studies with IAP inhibitor Debio 1143

Five groups (n=8) of adult female C57BL/6J mice (obtained from Shanghai Lingchang Bio-Technology Co.) were inoculated in the right lower flank with 1 × 10⁶ cells of the syngeneic colon carcinoma cell line MC38. When average tumor size reached about 50 mm³ (day 1), animals received either pretreatment consisting of p.o. SMAC mimetic Debio 1143 (Debiopharm) at a dose of 100 mg/kg or vehicle as indicated in Table 1. The dosing was repeated on each day for 7 days (day 1-7). On the subsequent day (day 8), animals of a vehicle-treated group and a Debio 1143-pretreated group were given i.p. 10 mg/kg of control antibody rlgG2b (Clone: LTF-2, BioXcell). Control antibody was administered twice weekly until the end of the study. Another set of two groups (vehicle- and Debio 1143-pretreated animals) received i.p. 10 mg/kg of anti-PD-L1 antibody (Mouse surrogate antibody, anti-mouse PD-L1, Clone: 10F.9G2, BioXcell). Administration was repeated twice weekly as for the control antibody. A final group of Debio 1143-pretreated animals received both anti-PD-L1 antibody as well as was continued on daily Debio 1143. Tumor volumes and body weights were assessed trice weekly. Tumor size was measured in two dimensions using a caliper, and the volume was expressed in mm³ using the formula: V = 0.5 a x *b*² where a and *b* are the long and short diameters of the tumor, respectively.

The results of the experiment are shown in Figure 4. Pretreatment with Debio 1143 alone (i.e., followed by administration of control antibody) had a modest anti-cancer effect (group 2). Treatment with PD-L1 antibody in the absence of a pretreatment with Debio 1143 essentially failed to retard tumor growth (group 3). The combination of a pretreatment with Debio 1143 followed by a treatment with PD-L1 antibody had a profound anti-cancer effect (group 4). Continuation of Debio 1143 during the treatment period appeared to provide a small additional benefit (group 5).

**Table 1: Experimental Design**

| **Group** | **n** | **Treatment** | | | | | | | |
|---|---|---|---|---|---|---|---|---|---|
| | | **Stage 1 (when mean TV @~50mm³, dosing from day 1 to day 7)** | | | | **Stage 2 (from day 8 to study end)** | | | |
| | | **Articles** | **Dose (mg/kg)** | **Dosing Route** | **Schedule** | **Articles** | **Dose (mg/kg)** | **Dosing Route** | **Schedule** |
| 1 | 8 | Vehicle of Debio1143 | - | p.o. | QD | rlgG2b | 10 | i.p. | BIW x 3wks |
| 2 | 8 | Debio1143 | 100 | p.o. | QD | rlgG2b | 10 | i.p. | BIW x 3wks |
| 3 | 8 | Vehicle of Debio1143 | - | p.o. | QD | anti-PD-L1 | 10 | i.p. | BIW x 3wks |
| 4 | 8 | Debio1143 | 100 | p.o. | QD | anti-PD-L1 | 10 | i.p. | BIW x 3wks |
| 5 | 8 | Debio1143 | 100 | p.o. | QD | anti-PD-L1 | 10 | i.p. | BIW x 3wks |
| | | | | | | Debio1143 | 100 | p.o. | QD x 21 days |

| | | | | | | | | | |
|---|---|---|---|---|---|---|---|---|---|
| p.o.: orally; i.p.: intraperitoneally; QD: daily; BIW: twice weekly | | | | | | | | | |

These animal studies provide direct evidence of the effectiveness of a pretreatment with an IAP antagonist to enhance the likelihood and/or the magnitude of an anti-tumor response to a subsequent treatment with an anti-PD-1 molecule.

### Example 3: IAP inhibitors birinapant and LCL161 pretreatment enhance efficacy of anti-PD-L1 in the MC38 model

72 adult female C57BL/6J mice (obtained from Shanghai Lingchang Bio-Technology Co.) were inoculated subcutaneously at the right lower flank with 1×10⁶ cells of the syngeneic colon carcinoma cell line MC-38 in 0.1 ml of PBS. Tumor volumes were measured three times weekly in two dimensions using a caliper, and the volume was expressed in mm³ using the formula: V = (L × W × W)/2, where V is tumor volume, L is tumor length (the longest tumor dimension) and W is tumor width (the longest tumor dimension perpendicular to L). All animals were randomly allocated to the 9 different study groups with a mean tumor size of 52 mm³ based on the "Matched distribution" randomization method (StudyDirectorTM software, version 3.1.399.19) and treatments started (denoted as day 1). Dosing as well as tumor and body weight measurement were conducted in a Laminar Flow Cabinet.

On day 1, part of the animals received either a 1 week pretreatment consisting of i.p. SMAC mimetic birinapant at a dose of 30 mg/kg, or its vehicle, in a biweekly schedule as indicated in Table 2. The other part of the animals received either a 1 week pretreatment consisting of p.o. SMAC mimetic LCL161 at a dose of 75 mg/kg, or its vehicle, in a biweekly schedule as indicated in Table 2.

On day 8, vehicle or SMAC mimetic pretreated animals were then further treated until study end with either biweekly i.p. 10 mg/kg of control antibody rlgG2b (Clone: LTF-2, BioXcell), or biweekly i.p. 10 mg/kg of anti-PD-L1 antibody (Mouse surrogate antibody, anti-mouse PD-L1, Clone: 10F.9G2, BioXcell). 1 group of animals that had received 1 week of birinapant pretreatment, and 1 group of animals that had received 1 week of LCL161 pretreatment, were each continued on the respective SMAC mimetic during the period of anti-PD-L1 treatment until study end.

The results of the experiment are shown in Figure 5 for birinapant, and Figure 6 for LCL161.

Pretreatment with birinapant alone (i.e., followed by administration of control antibody) had a modest anti-cancer effect (group 2). Treatment with anti-PD-L1 antibody in the absence of a pretreatment with birinapant essentially failed to retard tumor growth (group 3). The combination of a pretreatment with birinapant followed by a treatment with anti-PD-L1 antibody had a singificant anti-cancer effect (group 4). Continuation of birinapant during the treatment period appeared to provide a small additional benefit (group 5).

Pretreatment with LCL161 alone (i.e., followed by administration of control antibody) had a modest anti-cancer effect (group 7). The combination of a pretreatment with LCL161 followed by a treatment with anti-PD-L1 antibody appeared to provide a small additional benefit to LCL161 pretreatment alone (group 8), whereas continuation of LCL161 during the treatment period provided a significant additional benefit (group 9).

These animal studies provide direct evidence of the effectiveness of a pretreatment with any IAP antagonist to enhance the likelihood and/or the magnitude of an anti-tumor response to a subsequent treatment with an anti-PD-L1 molecule.

**Table 2: Experimental Design**

| **Group** | **N** | **Treatment** | | | | | | | |
|---|---|---|---|---|---|---|---|---|---|
| | | **Stage 1 (when mean TV @-50mm3, dosing start from day 1 to 7, one week)** | | | | **Stage 2 (from day 8 to study end)** | | | |
| | | **Articles** | **Dose (mg/kg)** | **Dosing Route** | **Schedule** | **Articles** | **Dose (mg/kg)** | **Dosing Route** | **Schedule** |
| **1** | 8 | Vehicle of Birinapant | - | i.p. | BIW | rlgG2b | 10 | i.p. | BIWx 3wks |
| **2** | 8 | Birinapant | 30 | i.p. | BIW | rlgG2b | 10 | i.p. | BIWx 3wks |
| **3** | 8 | Vehicle of Birinapant | - | i.p. | BIW | anti-PD-L1 | 10 | i.p. | BIWx 3wks |
| **4** | 8 | Birinapant | 30 | i.p. | BIW | anti-PD-L1 | 10 | i.p. | BIWx 3wks |
| **5** | 8 | Birinapant | 30 | i.p. | BIW | anti-PD-L1 | 10 | i.p. | BIWx 3wks |
| | | | | | | Birinapant | 30 | i.p. | BIWx 3wks |
| **6** | 8 | Vehicle of LCL 161 | - | p.o. | BIW | rlgG2b | 10 | i.p. | BIWx 3wks |
| **7** | 8 | LCL 161 | 75 | p.o. | BIW | rlgG2b | 10 | i.p. | BIWx 3wks |
| **8** | 8 | LCL 161 | 75 | p.o. | BIW | anti-PD-L1 | 10 | i.p. | BIWx 3wks |
| **9** | 8 | LCL 161 | 75 | p.o. | BIW | anti-PD-L1 | 10 | i.p. | BIWx 3wks |
| | | | | | | LCL161 | 75 | p.o. | BIWx 3wks |

| | | | | | | | | | |
|---|---|---|---|---|---|---|---|---|---|
| p.o.: orally; i.p.: intraperitoneally; QD: daily; BIW: twice weekly; wks: weeks. | | | | | | | | | |

### Example 4: 3.Debio 1143 induction enhances efficacy of anti-PD-1 in the CT26 model

Five groups (n=8) of adult female BALB/c mice (obtained from Shanghai Lingchang Bio-Technology Co.) were inoculated in the right lower flank with 0.5 × 10⁶ cells of the syngeneic colon carcinoma cell line CT26. When average tumor size reached about 50 mm³ (day 1), animals received either pretreatment consisting of p.o. SMAC mimetic Debio 1143 (Debiopharm) at a dose of 100 mg/kg or vehicle as indicated in Table 3a. The dosing was repeated on each day for 7 days (day 1-7). As indicated in Table 3b, on the subsequent day (day 8) animals of a vehicle-treated group and a Debio 1143-pretreated group were given daily oral vehicle until study end. Another set of two groups (vehicle- and Debio 1143-pretreated animals) received biweekly i.p. 10 mg/kg of anti-PD-1 antibody (Mouse surrogate antibody, anti-mouse PD-1, Clone: RMP1-14, BioXcell). A final group of Debio 1143-pretreated animals received both anti-PD-1 antibody as well as was continued on daily Debio 1143. Tumor volumes and body weights were assessed trice weekly. Tumor size was measured in two dimensions using a caliper, and the volume was expressed in mm³ using the formula: V = 0.5 a × *b*² where a and *b* are the long and short diameters of the tumor, respectively.

The results of the experiment are shown in Figure 7. Treatment with anti-PD-1 antibody in the absence of a pretreatment with Debio 1143 essentially failed to retard tumor growth (group 2). Pretreatment with Debio 1143 alone (i.e., followed by administration of oral vehicle) had a modest anti-cancer effect (group 3). The combination of a pretreatment with Debio 1143 followed by a treatment with anti-PD-1 antibody appeared to provide a small additional benefit to Debio 1143 pretreatment alone (group 4). Continuation of Debio 1143 during the treatment period provided a significant additional benefit (group 5).

These animal studies provide direct evidence of the effectiveness of a pretreatment with an IAP antagonist to enhance the likelihood and/or the magnitude of an anti-tumor response to a subsequent treatment with an anti-PD-1 molecule.

These animal studies provide direct evidence of the effectiveness of a pretreatment with any IAP antagonist to enhance the likelihood and/or the magnitude of an anti-tumor response to a subsequent treatment with any ICI molecule, in particular anti-PD-1 molecules, or anti-PD-L1 molecules.

**Table 3a. Pre-Treatment plan of the subcutaneous CT26 Colon Cancer Syngeneic Model in Female BALB/c mice**

| **Group** | **N** | **Treatment** | **Dose Level (mg/kg)** | **Dosing Solution (µg/µL)** | **Dosing Volume (µL/g)** | **Dosing route** | **Dosing Frequency** | **Schedule** |
|---|---|---|---|---|---|---|---|---|
| 1 | 8 | Vehicle | N/A | N/A | 10 | p.o. | QD | Day 1- 7 |
| 2 | 8 | Vehicle | N/A | N/A | 10 | p.o. | QD | Day 1- 7 |
| 3 | 8 | Debio 1143 | 100 | 10 | 10 | p.o. | QD | Day 1- 7 |
| 4 | 8 | Debio 1143 | 100 | 10 | 10 | p.o. | QD | Day 1- 7 |
| 5 | 8 | Debio 1143 | 100 | 10 | 10 | p.o. | QD | Day 1-7 |

| | | | | | | | | |
|---|---|---|---|---|---|---|---|---|
| p.o.: orally; i.p.: intraperitoneally; QD: daily; BIW: twice weekly; wks: weeks. | | | | | | | | |

**Table 3b. Continued treatment plan of the subcutaneous CT26 Colon Cancer Syngeneic Model in female BALB/c mice**

| **Grou P** | **N** | **Treatment** | **Dose Level (mg/kg)** | **Dosing Solution (µg/µL)** | **Dosing Volume (µL/g)** | **Dosing route** | **Dosing Frequency** | **Schedule** |
|---|---|---|---|---|---|---|---|---|
| 1 | 8 | Vehicle | N/A | N/A | 10 | p.o. | QD | From Day 8 |
| 2 | 8 | Anti-PD-1 | 10 | 1 | 10 | i.p. | BIW | From Day 8 |
| 3 | 8 | Vehicle | N/A | N/A | 10 | p.o. | QD | From Day 8 |
| 4 | 8 | Anti-PD-1 | 10 | 1 | 10 | i.p. | BIW | From Day 8 |
| 5 | 8 | Debio 1143 | 100 | 10 | 10 | p.o. | QD | From Day 8 |
| | | Anti-PD-1 | 10 | 1 | 10 | i.p. | BIW | From Day 8 |

| | | | | | | | | |
|---|---|---|---|---|---|---|---|---|
| p.o.: orally; i.p.: intraperitoneally; QD: daily; BIW: twice weekly; wks: weeks. | | | | | | | | |

### Scope and equivalence

Recitation of ranges of values herein are merely intended to serve as a shorthand method of referring individually to each separate value falling within the range, unless otherwise indicated herein, and each separate value is incorporated into the specification as if it were individually recited herein. Unless otherwise stated, all exact values provided herein are representative of corresponding approximate values (e. g., all exact exemplary values provided with respect to a particular factor or measurement can be considered to also provide a corresponding approximate measurement, modified by "about," where appropriate).

The use of any and all examples, or exemplary language (e.g., "such as") provided herein is intended merely to better illuminate the invention and does not pose a limitation on the scope of the invention unless otherwise indicated.

The citation of patent documents herein is done for convenience only and does not reflect any view of the validity, patentability and/or enforceability of such patent documents. The description herein of any aspect or embodiment of the invention using terms such as reference to an element or elements is intended to provide support for a similar aspect or embodiment of the invention that "consists of'," "consists essentially of" or "substantially comprises" that particular element or elements, unless otherwise stated or clearly contradicted by context (e. g. , a composition described as comprising a particular element should be understood as also describing a composition consisting of that element, unless otherwise stated or clearly contradicted by context).

## Claims

1. An inhibitor of apoptosis protein (IAP) antagonist for use in a method of treating cancer in a human subject, the method comprising:
(i) administering the IAP antagonist during an induction period, wherein the duration of the induction period is selected from the range of 1 to 48 days before first administration of an anti-PD-1 molecule; followed by
(ii) administering an anti-PD-1 molecule after the end of the induction period;
wherein:
the anti-PD-1 molecule is Nivolumab, Pembrolizumab, Atezolizumab, Durvalumab, Avelumab, PDR001, IBI-308, Cemiplimab, Camrelizumab, BGB-A317, BCD-100, JS-001, JNJ-3283, MEDI0680, AGEN-2034, TSR-042, Sym-021, PF-06801591, MGD-013, MGA-012, LZM-009, GLS-010, Genolimzumab, B! 754091, AK-104, CX-072, WBP3155, SHR-1316, PD-L1 Inhibitor millamolecule, BMS-936559, M-7824, LY-3300054, KN-035, FAZ-053, CK-301, or CA-170; and
the IAP antagonist administered during the induction period is Debio 1143, GDC-917/CUDC-427, LCL161, GDC-0152, TL-32711/Birinapant, HGS-1029/AEG-40826, B! 891065, ASTX-660 or APG-1387, preferably, the IAP antagonist is Debio 1143, LCL161 or Birinapant.

2. The IAP antagonist for use according to claim 1, wherein:
(a) the human subject is administered with the IAP antagonist during an induction period of 1 to 28 days, followed by the administration of the anti-PD-1 molecule;
(b) the human subject is administered with the IAP antagonist during an induction period of 5 to 28 days, followed by the administration of the anti-PD-1 molecule;
(c) the IAP antagonist is not administered on one or more days during the induction period; and/or
(d) the administration of the IAP antagonist is continued after the administration with the anti-PD-1 molecule has started, or another IAP antagonist is administered concurrently with the anti-PD-1 molecule.

3. The IAP antagonist for use according to claim 1 or 2, wherein:
(a) the cancer is a cancer that is known to be responsive to treatment with an anti-PD-1 molecule in 10% or more of treated patients;
(b) the cancer is head & neck cancer, melanoma, urothelial cancer, non-small cell lung cancer, microsatellite instability (MSI) high tumors from agnostic primary site or kidney cancer;
(c) the cancer is a cancer with a response rate to treatment with an anti-PD-1 molecule of 10% or less, preferably 5% or less; or
(d) the cancer is pancreatic cancer, colorectal cancer, multiple myeloma, small cell lung cancer, hepatocarcinoma or ovarian cancer.

4. The IAP antagonist for use according to any one of the preceding claims, wherein the cancer has been assessed to be poorly immunogenic.

5. The IAP antagonist for use according to claim 4, wherein said assessment consists of an analysis of a marker of immunogenicity in a patient's biological sample taken prior to the induction period and a finding that the marker's presence, expression level or derived score fails a predetermined threshold.

6. The IAP antagonist for use according to claim 5, wherein:
(a) the marker is PD-L1 expressed on cancer cells and/or immune cells; or
(b) the marker is tumor-infiltrating lymphocytes, preferably CD8+ cells, or tumor mutation burden.

7. The IAP antagonist for use according to any of the preceding claims, wherein the administering the IAP antagonist during an induction period is continued until the cancer is assessed to be of high immunogenicity.

8. The IAP antagonist for use according to claim 7, wherein said assessment consists of an analysis of a marker of immunogenicity in a patient's biological sample taken after the induction period and a finding that the marker's presence, expression level or derived score exceeds a predetermined threshold.

9. The IAP antagonist for use according to claim 8, wherein:
(a) the marker is PD-L1 expressed on cancer cells and/or immune cells; or
(b) the marker is tumor-infiltrating lymphocytes, preferably CD8+ cells, or tumor mutation burden.

10. The IAP antagonist for use according to claim 5, 6, 8 or 9, wherein the biological sample is a tumor or liquid biopsy.

11. The IAP antagonist for use according to any of the preceding claims, wherein the anti-PD-1 molecule is Nivolumab, Pembrolizumab, Atezolizumab, Durvalumab, Avelumab, PDR001, or B! 754091.

12. The IAP antagonist for use according to any one of claims 1 to 10, wherein the anti-PD-1 molecule is an antibody against PD-1 or PD-L1.

13. The IAP antagonist for use according to any one of the preceding claims, wherein the administration of the anti-PD-1 molecule is combined with one or more other cancer therapies, including another immunotherapy, radiotherapy, chemotherapy, chemioradiotherapy, oncolytic viruses, anti-angiogenic therapies, and/or targeted cancer therapies.

14. The IAP antagonist for use according to any one of the preceding claims, wherein the IAP antagonist is a second mitochondrial-derived activator of caspases (SMAC) mimetic.

15. The IAP antagonist for use according to any of the preceding claims , wherein the IAP antagonist is Debio 1143.

## Patentansprüche

1. Inhibitor von Apoptose-Protein (IAP)-Antagonist zur Verwendung in einem Verfahren zur Behandlung von Krebs in einem Menschen, das Verfahren umfassend:
(i) Verabreichung des LAP-Antagonisten während einer Induktionsperiode, wobei die Dauer der Induktionsperiode ausgewählt ist, aus dem Bereich von 1 bis 48 Tagen vor der ersten Verabreichung eines Anti-PD-1-Moleküls; gefolgt von
(ii) Verabreichung eines Anti-PD-1-Moleküls nach dem Ende der Induktionsperiode;
wobei:
das Anti-PD-1-Molekül Nivolumab, Pembrolizumab, Atezolizumab, Durvalumab, Avelumab, PDR001, IBI-308, Cemiplimab, Camrelizumab, BGB-A317, BCD-100, JS-001, JNJ-3283, MEDI0680, AGEN-2034, TSR-042, Sym-021, PF-06801591, MGD-013, MGA-012, LZM-009, GLS-010, Genolimzumab, BI 754091, AK-104, CX-072, WBP3155, SHR-1316, PD-L1 Inhibitor millamolecule, BMS-936559, M-7824, LY-3300054, KN-035, FAZ-053, CK-301, oder CA-170 ist; und
der während der Induktionsperiode verabreichte IAP-Antagonist Debio 1143, GDC-917/CUDC-427, LCL161, GDC-0152, TL-32711/Birinapant, HGS-1029/AEG-40826, BI 891065, ASTX-660 oder APG-1387 ist, vorzugsweise ist der IAP-Antagonist Debio 1143, LCL161 oder Birinapant.

2. IAP-Antagonist zur Verwendung gemäß Anspruch 1, wobei:
(a) dem Menschen der IAP-Antagonist während einer Induktionsperiode von 1 bis 28 Tage verabreicht wird, gefolgt von der Verabreichung des Anti-PD-1-Moleküls;
(b) dem Menschen der IAP-Antagonist während einer Induktionsperiode von 5 bis 28 Tage verabreicht wird, gefolgt von der Verabreichung des Anti-PD-1-Moleküls;
(c) der IAP-Antagonist während der Induktionsperiode an einem oder mehreren Tagen nicht verabreicht wird; und/oder
(d) die Verabreichung des LAP-Antagonisten wird nach Beginn der Verabreichung des Anti-PD-1-Moleküls fortgesetzt, oder ein anderer IAP-Antagonist wird gleichzeitig mit dem Anti-PD-1-Molekül verabreicht.

3. IAP-Antagonist zur Verwendung gemäß Anspruch 1 oder 2, wobei:
(a) der Krebs ein Krebs ist, von dem bekannt ist, dass er bei 10% oder mehr der behandelten Patienten auf die Behandlung mit einem Anti-PD-1-Molekül anspricht;
(b) der Krebs Kopf- und Halskrebs, Melanom, Urothelkarzinom, nicht-kleinzelliger Lungenkrebs, Mikrosatelliteninstabilität (MSI) hohe Tumoren von agnostischer Primärstelle oder Nierenkrebs ist;
(c) der Krebs ein Krebs ist, mit einer Ansprechrate auf die Behandlung mit einem Anti-PD-1-Molekül von 10% oder weniger, vorzugsweise 5% oder weniger; oder
(d) der Krebs Bauchspeicheldrüsenkrebs, kolorektales Karzinom, multiples Myelom, kleinzelliger Lungenkrebs, Hepatokarzinom oder Eierstockkrebs ist.

4. LAP-Antagonist zur Verwendung gemäß mindestens einem der vorhergehenden Ansprüche, wobei der Krebs als schwach immunogen bewertet wurde.

5. LAP-Antagonist zur Verwendung gemäß Anspruch 4, wobei die Bewertung aus einer Analyse eines Markers für Immunogenität in einer vor der Induktionsperiode entnommenen biologischen Probe eines Patienten und einer Feststellung, dass das Vorhandensein des Markers, das Expressionslevel oder der abgeleitete Score einen vorbestimmten Schwellenwert verfehlt, besteht.

6. IAP-Antagonist zur Verwendung gemäß Anspruch 5, wobei:
(a) der Marker PD-L1 exprimiert auf Krebszellen und/oder Immunzellen ist; oder
(b) der Marker tumorinfiltrierende Lymphozyten, vorzugsweise CD8+ Zellen, oder Tumormutationslast ist.

7. IAP-Antagonist zur Verwendung gemäß mindestens einem der vorhergehenden Ansprüche, wobei die Verabreichung des IAP-Antagonisten während einer Induktionsperiode fortgesetzt wird, bis der Krebs als hoch immunogen bewertet wird.

8. IAP-Antagonist zur Verwendung gemäß Anspruch 7, wobei die Bewertung aus einer Analyse eines Markers für Immunogenität in einer nach der Induktionsperiode entnommenen biologischen Probe eines Patienten und einer Feststellung, dass das Vorhandensein des Markers, das Expressionslevel oder der abgeleitete Score einen vorbestimmten Schwellenwert überschreitet, besteht.

9. IAP-Antagonist zur Verwendung gemäß Anspruch 8, wobei:
(a) der Marker PD-L1 exprimiert auf Krebszellen und/oder Immunzellen ist; oder
(b) der Marker tumorinfiltrierende Lymphozyten, vorzugsweise CD8+ Zellen, oder Tumormutationslast ist.

10. IAP-Antagonist zur Verwendung gemäß Anspruch 5, 6, 8 oder 9, wobei die biologische Probe ein Tumor oder eine Flüssigbiopsie ist.

11. IAP-Antagonist zur Verwendung gemäß mindestens einem der vorhergehenden Ansprüche, wobei das Anti-PD-1-Molekül Nivolumab, Pembrolizumab, Atezolizumab, Durvalumab, Avelumab, PDR001, oder BI 754091 ist.

12. IAP-Antagonist zur Verwendung gemäß mindestens einem der Ansprüche 1 bis 10, wobei das Anti-PD-1-Molekül ein Antikörper gegen PD-1 oder PD-L1 ist.

13. IAP-Antagonist zur Verwendung gemäß mindestens einem der vorhergehenden Ansprüche, wobei die Verabreichung des Anti-PD-1-Moleküls mit einer oder mehreren anderen Krebstherapien kombiniert wird, einschließlich einer anderen Immuntherapie, Strahlentherapie, Chemotherapie, Radiochemotherapie, onkolytischen Viren, antiangiogenen Therapien, und/oder gezielten Krebstherapien.

14. IAP-Antagonist zur Verwendung gemäß mindestens einem der vorhergehenden Ansprüche, wobei der IAP-Antagonist ein zweites Mitochondrien-abgeleitetes Aktivator von Caspasen (SMAC)-Mimetikum ist.

15. IAP-Antagonist zur Verwendung gemäß mindestens einem der vorhergehenden Ansprüche, wobei der IAP-Antagonist Debio 1143 ist.

## Revendications

1. Inhibiteur de l'antagoniste de la protéine d'apoptose (IAP) pour une utilisation dans un procédé de traitement d'un cancer chez un sujet humain, le procédé comprenant :
(i) l'administration de l'antagoniste de l'IAP pendant une période d'induction, dans lequel la durée de la période d'induction est choisie dans la plage de 1 à 48 jours avant la première administration d'une molécule anti-PD-1 ; suivie de
(ii) l'administration d'une molécule anti-PD-1 après la fin de la période d'induction ;
dans lequel :
la molécule anti-PD-1 est Nivolumab, Pembrolizumab, Atezolizumab, Durvalumab, Avelumab, PDR001, IBI-308, Cemiplimab, Camrelizumab, BGB-A317, BCD-100, JS-001, JNJ-3283, MEDI0680, AGEN-2034, TSR-042, Sym-021, PF-06801591, MGD-013, MGA- 012, LZM-009, GLS-010, Genolimzumab, BI 754091, AK-104, CX-072, WBP3155, SHR-1316, une millamolécule d'inhibiteur de PD-L1 , BMS-936559, M-7824, LY-3300054, KN-035, FAZ-053, CK-301 ou CA-170 ; et
l'antagoniste de l'IAP administré pendant la période d'induction est Debio 1143, GDC- 917/CUDC-427, LCL161, GDC-0152, TL-32711/Birinapant, HGS-1029/AEG-40826, BI 891065, ASTX-660 ou APG-1387, de préférence, l'antagoniste de l'IAP est Debio 1143, LCL161 ou Birinapant.

2. Antagoniste de l'IAP pour une utilisation selon la revendication 1, dans lequel :
(a) le sujet humain reçoit une administration de l'antagoniste de l'IAP pendant une période d'induction de 1 à 28 jours, suivie de l'administration de la molécule anti-PD-1;
(b) le sujet humain reçoit une administration de l'antagoniste de l'IAP pendant une période d'induction de 5 à 28 jours, suivie de l'administration de la molécule anti-PD-1;
(c) l'antagoniste de l'IAP n'est pas administré pendant un ou plusieurs jours pendant la période d'induction ; et/ou
(d) l'administration de l'antagoniste de l'IAP est poursuivie après que l'administration de la molécule anti-PD-1 a démarré, ou un autre antagoniste de l'IAP est administré en même temps que la molécule anti-PD-1.

3. Antagoniste de l'IAP pour une utilisation selon la revendication 1 ou 1, dans lequel :
(a) le cancer est un cancer qui est connu pour répondre à un traitement par une molécule anti-PD-1 chez 10 % ou plus des patients traités ;
(b) le cancer est un cancer de la tête et du cou, un mélanome, un cancer urothélial, un cancer du poumon non à petites cellules, des tumeurs à instabilité des microsatellites (MSI) élevée provenant d'un site primaire agnostique ou un cancer du rein ;
(c) le cancer est un cancer présentant un taux de réponse à un traitement par une molécule anti-PD-1 de 10 % ou moins, de préférence de 5 % ou moins ; ou
(d) le cancer est un cancer du pancréas, un cancer colorectal, un myélome multiple, un cancer du poumon à petites cellules, un hépatocarcinome ou un cancer de l'ovaire.

4. Antagoniste de l'IAP pour une utilisation selon l'une quelconque des revendications précédentes, dans lequel le cancer a été évalué comme étant peu immunogène.

5. Antagoniste de l'IAP pour une utilisation selon la revendication 4, dans lequel ladite évaluation est constituée d'une analyse d'un marqueur de l'immunogénicité dans un échantillon biologique d'un patient prélevé avant la période d'induction et d'une découverte que la présence, le niveau d'expression ou le score dérivé du marqueur ne satisfait pas un seuil prédéterminé.

6. Antagoniste de l'IAP pour une utilisation selon la revendication 5, dans lequel :
(a) le marqueur est le PD-L1 exprimé sur des cellules cancéreuses et/ou des cellules immunitaires ; ou
(b) le marqueur est des lymphocytes infiltrant les tumeurs, de préférence des cellules CD8+, ou une charge de mutation tumorale.

7. Antagoniste de l'IAP pour une utilisation selon l'une quelconque des revendications précédentes, dans lequel l'administration de l'antagoniste de l'IAP pendant une période d'induction est poursuivie jusqu'à ce que le cancer soit évalué comme étant d'immunogénicité élevée.

8. Antagoniste de l'IAP pour une utilisation selon la revendication 7, dans lequel ladite évaluation consiste en une analyse d'un marqueur de l'immunogénicité dans un échantillon biologique d'un patient prélevé après la période d'induction et une découverte que la présence, le niveau d'expression ou le score dérivé du marqueur dépasse un seuil prédéterminé.

9. Antagoniste de l'IAP pour une utilisation selon la revendication 8, dans lequel :
(a) le marqueur est le PD-L1 exprimé sur des cellules cancéreuses et/ou des cellules immunitaires ; ou
(b) le marqueur est des lymphocytes infiltrant les tumeurs, de préférence des cellules CD8+, ou une charge de mutation tumorale.

10. Antagoniste de l'IAP pour une utilisation selon la revendication 5, 6, 8 ou 9, dans lequel l'échantillon biologique est une tumeur ou une biopsie liquide.

11. Antagoniste de l'IAP pour une utilisation selon l'une quelconque des revendications précédentes, dans lequel la molécule anti-PD-1 est Nivolumab, Pembrolizumab, Atezolizumab, Durvalumab, Avelumab, PDR001 ou BI 754091.

12. Antagoniste de l'IAP pour une utilisation selon l'une quelconque des revendications 1 à 10, dans lequel la molécule anti-PD-1 est un anticorps contre PD-1 ou PD-L1.

13. Antagoniste de l'IAP pour une utilisation selon l'une quelconque des revendications précédentes, dans lequel l'administration de la molécule anti-PD-1 est combinée à une ou plusieurs autres thérapies contre le cancer, y compris une autre immunothérapie, une radiothérapie, une chimiothérapie, une chimioradiothérapie, des virus oncolytiques, des thérapies anti-angiogéniques et/ou des thérapies anticancéreuses ciblées.

14. Antagoniste de l'IAP pour une utilisation selon l'une quelconque des revendications précédentes, dans lequel l'antagoniste de l'IAP est un second activateur dérivé des mitochondries de caspases (SMAC) mimétique.

15. Antagoniste de l'IAP pour une utilisation selon l'une quelconque des revendications précédentes, dans lequel l'antagoniste de l'IAP est Debio 1143.
